# EUROPEAN PATENT APPLICATION

(11) **EP 4 733 319 A1**
(43) Date of publication of application: **29.04.2026**
(21) Application number: 24306805.3
(22) Date of filing: 25.10.2024
(51) Int. Cl.: C07K 16/24, A61K 39/395

(54) **THERAPEUTIC MOLECULES**

(71) Applicant: Kyron.bio, 75008 Paris (FR)
(72) Inventor: MCLAUGHLIN, Emilia Jane, 75008 PARIS (FR); LOCHHEAD, Marina Rose, 75008 PARIS (FR)
(74) Representative: Appleyard Lees IP LLP

(57) **Abstract**

The present invention relates to methods for engineering the variable region of antibodies or antigen binding fragments thereof, in particular to reduce the effect of anti-drug antibodies. In one aspect, there is provided a monoclonal antibody or antigen-binding fragment thereof, wherein an amino acid sequence of the antibody or fragment thereof is engineered to reduce immunogenicity of the antibody or fragment thereof when the antibody or fragment thereof is administered to a subject, wherein the amino acid sequence is engineered to comprise one or more N-X-S and/or N-X-T sequence motifs in a variable region of the monoclonal antibody or fragment thereof, and wherein X is any amino acid other than proline.

## Description

### Field of the invention

The present invention relates to engineered/modified antibodies, particularly antibodies with a modified variable region and uses thereof.

### Background

Monoclonal antibodies (mAbs) represent a major class of protein therapeutics, accounting for more than 50% of all new approvals in the last few years (Walsh et al, 2022). With diverse indication areas including cancer, infection, and inflammatory and autoimmune diseases, they are indeed the fastest growing sector of biologics in the pharmaceutical industry (Torre et al, 2023). Despite the clinical success of mAbs, challenges in their development and therapeutic activity remain, most notably of which is the immune responses that are triggered upon therapeutic administration to patients. Immune responses are observed in response to non-human sequences, predominantly murine-derived, that are found in the mAb protein therapeutic. Seminal work to deimmunize mAb therapeutics was the humanization of mAbs which replaces non-human sequences with human ones. This process generates mAbs made up of almost entirely human sequences, except for the complementarity-determining region (CDR), the portion of the antibody that binds its target, which remains murine-derived. This strategy of humanization can reduce immunogenicity but does not eliminate it all together. Indeed, even some fully human mAbs with human sequences in both the CDR and the rest of the antibody structure still elicit unwanted immune responses (Wang et al, 2016).

The immunogenic potential of mAbs mainly manifests in anti-drug antibody (ADA) generation (Hwang et al, 2005; Winter et al, 1994; Vaughan et al, 1996). The presence of ADAs has significant clinical impact, altering the drug's pharmacokinetic (PK) and pharmacodynamic (PD) properties, and lowering serum half-life and bioavailability, thereby ultimately reducing drug efficacy (Atzeni et al, 2013; De Groot et al, 2007; Hansel et al, 2010). Additionally, ADAs have a significant impact on the drug safety, leading to serious adverse immune responses in patients and longer non-remission times (de Vries et al, 2007; Yanai et al, 2011). ADAs are emerging as a major clinical hurdle in newly developed biotherapeutics including bispecific antibodies and multi-specific products that are entering the clinic, such as cancer immunotherapies (Zhou et al, 2022). Despite hundreds of bispecifics in clinical development, only a handful have been approved, with failed bispecifics highlighting immunogenicity as one of the key challenges in development. These atypical antibodies contain highly engineered sequences that can trigger immunogenic responses (Ma et al, 2021). Therefore, the present invention represents a significant contribution to the art. The inventors' targeted structure-based antibody engineering strategy could also be applied to therapeutics outside of traditional monoclonal antibody therapeutics that are otherwise hindered by the development of anti-drug antibodies.

ADAs can be categorized into two types of antibodies: neutralizing ADAs which associate with the target binding site in the CDR of the therapeutic and prevent target binding, thereby inhibiting the biological activity of the therapeutic, and non-neutralizing ADAs which bind outside of the target binding site and do not inhibit the biological activity of the therapeutic but still impact the PK and PD of the drug (Shankar et al, 2014; Krieckaert et al, 2010). Although neutralizing ADAs are more problematic as they block the therapeutic mode of action, both types of ADAs can increase the clearance of the protein therapeutic from circulation, reducing efficacy (Liu et al, 2018).

Therefore, it can be seen that there is a need to develop antibodies which are resistant to the effect of ADAs while also maintaining their binding capacity so as to achieve their intended therapeutic effect.

### Summary

The inventors have surprisingly shown that the binding of ADAs to an antibody or fragment thereof can be reduced by glycosylating one or more residue in a variable region of an antibody or antigen binding fragment thereof that is involved in the binding of antigen or located in proximity thereto in a variable region of an antibody or antigen binding fragment thereof. Glycosylation of such residue is predicted to result in reduced ADA binding, ADA response, immunogenicity and/or unwanted side effects of the antibody or antigen binding fragment thereof in medical treatments. Less ADA binding results in reduced clearance. In particular and surprisingly, the inventors have shown that ADA binding can be reduced by glycosylating one or more residues in a CDR1, 2 and/or 3 region on the heavy and/or light chain of an antibody that binds to ADAs. The inventors have also shown that such modification can be made without /without substantially impacting the therapeutic potential of the antibody, i.e. binding to target.

Thus, the inventors have surprisingly found that certain modifications to introduce glycosylation sites can be successfully made to a target sequence of the variable region, including the CDR, of antibodies, which enables the antibodies to maintain their high specificity and affinity to their target and at the same time reduce ADA binding to the antibody. Such antibodies may therefore benefit from reduced ADA generation and reduced ADA binding to the antibody when administered to a subject while still achieving their desired binding/therapeutic effect. Even more surprisingly, the inventors of the present invention have identified mutations and modifications that can be made within the CDR regions are particularly effective at reducing ADA binding, while maintaining target binding activity.

In one aspect, the invention relates to an antibody or antigen binding fragment thereof with reduced ADA binding comprising a modified amino acid sequence in the variable region wherein said amino acid sequence is modified to comprise one or more N-X-S and/or N-X-T sequence motifs wherein X is any amino acid other than proline.

In one embodiment, a CDR1, 2 or 3 region or a region within 20, e.g. 10 amino acid residues of a framework region adjacent to a CDR1, 2 or 3 region is modified.

In one embodiment, one or more amino acid residue located in a variable region is substituted with N, T or S.

In one embodiment, a N-X-S/T sequence motif is inserted into an amino acid residue sequence.

In one embodiment, a CDR1, 2 or 3 region or a region or within 1, 2, 3, 4 or 5 amino acid residues of a framework region adjacent to a CDR1, 2 or 3 region is modified.

In one embodiment, a CDR1, 2 or 3 region is modified.

In one embodiment, said one or more motif is located in an epitope for binding ADAs or in proximity thereto.

In one embodiment, the one or more amino acid residue is substituted with N.

In one embodiment, the one or more amino acid residue is substituted with T.

In one embodiment, the one or more amino acid residue is replaced with S.

In one embodiment, said variable region is a heavy chain variable region or light chain variable region.

In one embodiment, the epitope is a linear epitope or a confirmational epitope.

In one embodiment, said fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.

In one embodiment, said antibody or fragment is conjugated to another moiety.

In one embodiment, the other moiety is a therapeutic moiety, half-life extending moiety, toxin or label.

In one embodiment, said antibody or antigen binding fragment thereof is N-glycosylated at the N-X-S/T sequence motif.

In another aspect, the invention relates to a nucleic acid encoding an antibody or antigen binding fragment thereof as described above.

In another aspect, the invention relates to a vector comprising a nucleic acid as described above.

In another aspect, the invention relates to a host cell comprising a nucleic acid or a vector according as described above.

In another aspect, the invention relates to a pharmaceutical composition comprising an antibody or antigen binding fragment thereof as described above and a pharmaceutically acceptable excipient.

In another aspect, the invention relates to an antibody or antigen binding fragment thereof according to as described above or a pharmaceutical composition as described above in the treatment of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases.

In another aspect, the invention relates to a method of treating of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases comprising administering antibody or antigen binding fragment thereof as described above or a pharmaceutical composition as described above to a subject.

In one embodiment, the antibody or antigen binding fragment is administered together with another therapy. In another aspect, the invention relates to a method for producing an antibody or antigen binding fragment thereof with reduced ADA binding comprising modifying the amino acid sequence of the variable region to comprise one or more N-X-S and/or N-X-T sequence motifs wherein X is any amino acid other than proline. In one embodiment, said one or more motif is located in an epitope for antigen binding or in proximity thereto. In one embodiment, one or more amino acid residue located in a variable region is substituted with N, T or S to introduce a N-X-S/T sequence motif.

In one embodiment, a N-X-S/T sequence motif is inserted into an amino acid residue sequence located in an epitope for antigen binding or in proximity thereto.

In one embodiment, the method comprises the steps of
a) substituting one or more amino acid residue in a variable region with a N, S or T to introduce a N-X-S/T sequence motif and wherein said one or more amino acid residue is located in an epitope for antigen binding and
b) expressing a nucleotide sequence or nucleic acid encoding the modified antibody or antigen binding fragment thereof in a suitable host cell or host organism.

In another aspect, the invention relates to a method for reducing the ADA response of an antibody or antigen binding fragment thereof with reduced immunogenicity comprising modifying the amino acid sequence of the variable region to comprise one or more N-X-S and/or N-X-T sequence motifs wherein X is any amino acid other than proline.

In one embodiment, said one or more motif is located in an epitope for antigen binding or in proximity thereto.

In one embodiment, one or more amino acid residue located in a variable region is substituted with N, T or S to introduce a N-X-S/T sequence motif.

In one embodiment, a N-X-S/T sequence motif is inserted into an amino acid residue sequence located in an epitope for antigen binding or in proximity thereto.

In one embodiment, the method comprises the steps of
a) substituting one or more amino acid residue in a variable region with a N, S or T to introduce a N-X-S/T sequence motif and wherein said one or more amino acid residue is located in an epitope for antigen binding and
b) expressing a nucleotide sequence or nucleic acid encoding the modified antibody or antigen binding fragment thereof in a suitable host cell or host organism.

In one embodiment, said variable region is a heavy chain variable region or light chain variable region.

In one embodiment, the one or more amino acid residue is located in a CDR1, 2 or 3 region or within 20, e.g.10 amino acid residues of a framework region adjacent to a CDR1 , 2 or 3 region.

In one embodiment, the one or more amino acid residue is located in a CDR1, 2 or 3 region or within 1, 2, 3, 4 or 5 amino acid residues of a framework region adjacent to a CDR1, 2 or 3 region.

In one embodiment, the one or more amino acid residue is located in a CDR1, 2 or 3 region.

In one embodiment, the one or more amino acid residue is substituted with N.

In one embodiment, the one or more amino acid residue is substituted with T.

In one embodiment, the one or more amino acid residue is substituted with S.

In one embodiment, the method comprises the step of assessing binding of the antibody or antigen binding fragment thereof to its target antigen and/or assessing binding ADAs to the antibody or antigen binding fragment thereof.

In one embodiment, the method comprises the step of determining which one or more residue in the variable region is located in an epitope for antigen binding.

In one embodiment, the method comprises the step of determining which one or more residue in the variable region is located in an epitope for ADA binding.

In one embodiment,the epitope is a linear epitope or a confirmational epitope.

In one embodiment,said fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.

In another aspect, the invention relates to a kit comprising antibody or antigen binding fragment thereof as described above and optionally instructions for use.

In another aspect, the invention relates to a binding molecule comprising antibody or antigen binding fragment thereof as described above.

In one embodiment, the binding molecule comprises a second antibody or antigen binding fragment thereof which binds to the same or a different target.

### Figures

The invention is further described in the following non-limiting figures.
FIGURE 1. Adalimumab glycovariant design showing 4 separate sites identified that can each individually be mutated to an Asn in order to generate an N-X-S/T sequon in the CDR region of adalimumab that blocks ADA interaction. Four separate adalimumab variants were generated: W53N, Y101N, L102N, and A105N.
FIGURE 2. Expression of adalimumab glycovariants in different CHO cells.
FIGURE 3. PNGase F assay showing representative data showing that the change in MW is a result of additional N-glycans expressed a CHO cell.
FIGURE 4. TNFα binding assay showing representative data showing the adalimumab variant Y101N interacting with soluble TNFα.
FIGURE 5. A) ADA binding assay showing complete disruption of monoclonal ADAs binding to W53N, Y101N, L102N, and A105N variants of adalimumab. Monoclonal ADAs were added to every condition. B) ADA binding assay showing complete disruption of monoclonal ADAs binding to Y101N and sustained binding of monoclonal ADAs with Y101A and Y101Q mutations indicating the presence of the N-glycan at this site is crucial for disrupting ADA binding. Monoclonal ADAs were added to every condition.
FIGURE 6. ADA binding assay showing disruption of L102N variant binding to polyclonal ADAs.
FIGURE 7. ELISA-based out-competition assay carried out using serum from A) ankylosing spondylitis patients and B) and C) rheumatoid arthritis patients. Results show that the adalimumab variant L102N represented in orange significantly reduces unwanted immune clearance compared to market leader Humira^{®} (adalimumab) shown in black. Error bars, S.D. (n = 3).

### Detailed description of the embodiments

The embodiments of the invention will now be further described. In the following passages, different embodiments are described. Each aspect so defined may be combined with any other aspect or aspects unless clearly indicated to the contrary. In particular, any feature indicated as being preferred or advantageous may be combined with any other feature or features indicated as being preferred or advantageous.

Generally, nomenclatures used in connection with, and techniques of, cell and tissue culture, pathology, oncology, molecular biology, immunology, microbiology, genetics and protein and nucleic acid chemistry and hybridization described herein are those well-known and commonly used in the art. The methods and techniques of the present disclosure are generally performed according to conventional methods well-known in the art and as described in various general and more specific references that are cited and discussed throughout the present specification unless otherwise indicated. See, e.g., Green and Sambrook et al., Molecular Cloning: A Laboratory Manual, 4th ed., Cold Spring Harbor Laboratory Press, Cold Spring Harbor, N.Y. (2012); Therapeutic Monoclonal Antibodies: From Bench to Clinic, Zhiqiang An (Editor), Wiley, (2009); and Antibody Engineering, 2nd Ed., Vols 1 and 2, Kontermann and Duebel, eds., Springer-Verlag, Heidelberg (2010).

Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients. Suitable assays to measure the properties as set out above are also described in the examples.

The inventors of the present invention have in particular found that the impact of ADAs can be reduced by engineering, i.e. modifying certain amino acid sequence motifs into the variable region of antibodies, particularly the CDRs, which allow for their subsequent modification. This subsequent modification may be referred to as a co-translational and/or post-translational modification, and within the context of the present invention refers to co- and/or post-translational N-linked glycosylation.

In all aspects of the invention, "engineered" or "modified" refers to any amino acid modification of a target sequence, e.g. a reference or wild type sequence. Modification may be the substitution of a reference amino acid residue with another amino acid residue, insertion of one or more amino acid into a reference sequence or deletion of one or more amino acid. Sequence motifs as described herein may be introduced into the amino acid sequence of the antibody or antigen binding fragment thereof by several routine methods known to those in the art.

In a first aspect, the invention relates to an antibody or antigen binding fragment thereof with reduced ADA binding comprising a modified amino acid sequence in the variable region wherein said amino acid sequence is modified to comprise one or more N-X-S and/or N-X-T sequence motifs wherein X is any amino acid other than proline.

In one embodiment, said one or more motif is located in an epitope for antigen binding or in proximity thereto.

In one embodiment, one or more amino acid residue located in a variable region is substituted with N, T or S to introduce a N-X-S/T sequence motif and wherein said amino acid residue is located in an epitope for antigen binding or in proximity thereto.

In one embodiment, a N-X-S/T sequence motif is inserted into an amino acid residue sequence located in an epitope for antigen binding or in proximity thereto.

In one embodiment, one or more amino acid residue located in the variable region is substituted with N, T or S to introduce a N-X-S/T sequence motif wherein said amino acid residue is located in an epitope for antigen binding or in proximity thereto.

The term "in proximity" thereto refers to any amino acid that is close to the epitope, e.g. referring to the linear amino acid sequence, within 20, e.g. 10 amino acid residues; e.g. 1 to 10 amino acid residues, adjacent to the amino acid residue located in the epitope (i.e. the 20 e.g. 10 residues located N or C terminally of the respective residue) that forms part of the epitope for antigen binding. "Adjacent to" includes residues either side, i.e. N or C terminally. The term may also refer to residues that are in close conformational proximity to the epitope.

Thus, an amino acid sequence of the antibody or fragment thereof is engineered by altering a target sequence, e.g. a reference, e.g. a wild type amino acid residue to introduce a N-X-S/T sequence motif, thereby reducing ADA binding and/or immunogenicity of the antibody or fragment thereof when the antibody or fragment thereof is administered to a subject. Target sequence refers to the antibody sequence which is modified. This can also be described as a reference sequence. Reference sequence thus refers to the amino acid sequence that is being altered. Wild type refers to the native residue or sequence of the antibody or fragment thereof. Reference sequence may refer to the native, i.e. wild type sequence, but a skilled person would know that it can also encompass a sequence of the antibody or fragment thereof that has also been engineered in other ways to achievable other desirable outcomes and thus no longer has a native sequence.

The antibody or fragment thereof of the invention exhibits reduced ADA binding and/or immunogenicity when administered to an individual, e.g. an individual that is receiving treatment with said antibody or antigen binding fragment thereof.

'X' may be any canonical or non-canonical amino acid other than proline. A canonical amino acid may be any of the 20 naturally occurring amino acids which are listed herein. A non-canonical amino acid may be any amino acid that is not one of the 20 naturally occurring amino acids. Non-canonical amino acids may arise from chemical modifications (which may occur by synthetic or natural means) to naturally occurring amino acids.

The term "antibody" as used herein broadly includes, but is not limited to, any immunoglobulin (Ig) molecule, or antigen binding portion thereof, comprised of four polypeptide chains, two heavy (H) chains and two light (L) chains, or any functional fragment, mutant, variant, or derivation thereof, which retains the essential epitope binding features of an Ig molecule.

In a full-length antibody, each heavy chain is comprised of a heavy chain variable region or domain (abbreviated herein as HCVR) and a heavy chain constant region. The heavy chain constant region is comprised of three domains, C_{H}1, C_{H}2 and C_{H}3. Each light chain has a light chain variable region or domain (abbreviated herein as LCVR) and a light chain constant region. The light chain constant region is comprised of one domain, C_{L}.

The heavy chain and light chain variable regions can be further subdivided into regions of hypervariability, termed complementarity determining regions (CDR), interspersed with regions that are more conserved, termed framework regions (FR). Each heavy chain and light chain variable region are composed of three CDRs and four FRs, arranged from amino-terminus to carboxy-terminus in the following order: FR1, CDR1, FR2, CDR2, FR3, CDR3, FR4.

Immunoglobulin molecules can be of any type (e.g., IgG, IgE, IgM, IgD, IgA and IgY), class (e.g., IgG 1, IgG2, IgG 3, IgG4, IgAl and IgA2) or subclass.

The term "CDR" or "CDR region" refers to the complementarity-determining region within antibody variable sequences. There are three CDRs in each of the variable regions of the heavy chain and the light chain, which are designated CDR1, CDR2 and CDR3, for each of the variable regions. The term "CDR set" refers to a group of three CDRs that occur in a single variable region capable of binding the antigen. The exact boundaries of these CDRs can be defined differently according to different systems known in the art.

The Kabat Complementarity Determining Regions (CDRs) are based on sequence variability and are the most commonly used (Kabat et al., (1971) Ann. NY Acad. Sci. 190:382-391 and Kabat, et al., (1991) Sequences of Proteins of Immunological Interest, Fifth Edition, U.S. Department of Health and Human Services, NIH Publication No. 91-3242). Chothia refers instead to the location of the structural loops (Chothia and Lesk J. Mol. Biol. 196:901 -917 (1987)). The Kabat numbering system is generally used when referring to a residue in the variable domain (approximately residues 1-107 of the light chain and residues 1 -113 of the heavy chain). Another system is the ImMunoGeneTics (IMGT) numbering scheme. The IMGT numbering scheme is described in Lefranc et al., Dev. Comp. Immunol., 29, 185-203 (2005).

The system described by Kabat is used herein unless otherwise mentioned, but alternatively IMGT or other numbering systems can be used. The terms "Kabat numbering", "Kabat definitions" and "Kabat labelling" are used interchangeably herein. These terms, which are recognized in the art, refer to a system of numbering amino acid residues which are more variable (i.e., hypervariable) than other amino acid residues in the heavy and light chain variable regions of an antibody, or an antigen binding portion.

The antibody may be human, humanized or chimeric. In one embodiment, the antibody may be canine or feline.

A "chimeric antibody" is a recombinant protein that contains the variable domains including the complementarity determining regions (CDRs) of an antibody derived from one species, preferably a rodent antibody, while the constant domains of the antibody molecule are derived from those of a human antibody.

A "humanised antibody" is a recombinant protein in which the CDRs from an antibody from one species; e.g., a rodent antibody, are transferred from the heavy and light variable chains of the rodent antibody into human heavy and light variable domains (e.g., framework region sequences). The constant domains of the antibody molecule are derived from those of a human antibody. In certain embodiments, a limited number of framework region amino acid residues from the parent (rodent) antibody may be substituted into the human antibody framework region sequences.

Humanisation is typically undertaken since non-human antibodies will evoke an undesirable immune reaction when administered to a human subject. Non-human sequences may be derived from, but are not limited to, murine-derived antibodies, porcine-derived antibodies, bovine-derived antibodies, ovine-derived antibodies, simian-derived antibodies, leporine-derived antibodies, feline-derived antibodies, canine-derived antibodies, ursine-derived antibodies, piscine-derived antibodies and so on. The process of humanising antibodies most often involves the use of recombinant DNA techniques with which the skilled person will be familiar.

As used herein, the term "monoclonal antibody" or "mAb" is given its usual meaning in the art, and refers to a monospecific antibody that is produced from homogeneous cells that are each clones of the same origin parent cell or cell line. The resultant antibodies are identical in sequence and therefore bind to the same epitope. In contrast to monoclonal antibodies, polyclonal antibodies (pAbs) are made from several different immune cells and have affinity for the same antigen but different epitopes. Monoclonal antibodies require production in laboratories whereas polyclonal antibodies can be found naturally, for example within the human body. In one embodiment, the antibodies of the present invention may be monoclonal antibodies.

As used herein, the term "antigen binding region" as used herein refers to an antibody, antigen binding fragment thereof or antibody mimetic.

The term "antigen binding site" refers to the part of the antibody or antibody fragment that comprises the area that specifically binds to an antigen. An antigen binding site may be provided by one or more antibody variable domains. An antigen binding site is typically comprised within the associated V_{H} and V_{L} of an antibody or antibody fragment.

The term antibody as used herein also includes antibody fragments. Specifically, the invention also extends to antibody fragments. Antibody fragments are functional fragments of a full-length antibody, that is they retain the target specificity of a full antibody.

An antibody fragment is a portion of an antibody, for example a Fab (Fragment, antibody), F(ab')2, Fv, scFv (single chain variable chain fragments), heavy chain, light chain, variable heavy (VH) chain, variable light (VL) chain, CDR region, single VH or single VL domain, single VHH domain (nanobody), maxibodies, minibodies, intrabodies, diabodies, triabodies, tetrabodies, and bis-scFv, and polypeptides that contain at least a portion of an immunoglobulin that is sufficient to confer specific antigen binding to the polypeptide. Therefore, an antibody fragment comprises an antigen binding portion.

Thus, in one embodiment, the fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.

An "Fv" is the minimum antibody fragment which contains a complete antigen- recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

"Single-chain Fv" also abbreviated as "sFv" or "scFv" fragments (~25kDa) comprise of the two variable domains, V_{H} and V_{L} connected into a single polypeptide chain. Naturally, V_{H} and V_{L} domain are non-covalently associated via hydrophobic interaction and tend to dissociate. However, stable fragments can be engineered by linking the domains with a hydrophilic flexible linker to create a single chain Fv (scFv).

A diabody is a non-covalent dimer of a scFv fragment comprising the heavy chain variable region and light chain variable region connected by a small peptide linker. A triabody is a trimer of ScFvs, and a tetrabody is a tetramer of ScFvs.

A minibody is composed a single pair of ScFvs which are linked via a disulphide bond in a constant region of the heavy chain (known as the CH3 domain). Minibodies may be monospecific or bispecific. Further, a minibody may be generated by the addition of a third binding domain onto, for example, the constant region of the minibody.

The smallest antigen binding fragment is the single variable fragment, namely the single variable heavy (V_{H}) or single variable light (V_{L}) chain domain. V_{H} and V_{L} domains respectively are capable of binding to an antigen. Binding to a light chain/heavy chain partner respectively or indeed the presence of other parts of the full antibody is not required for target binding. The antigen-binding entity of an antibody, reduced in size to one single domain (corresponding to the V_{H} or V_{L} domain), is generally referred to as a "single domain antibody" or "single immunoglobulin variable domain". A single domain antibody (~12 to 15 kDa) thus consists of either the V_{H} or V_{L} domain, but it does not comprise other parts of a full-length antibody. Single domain antibodies derived from camelid heavy chain only antibodies that are naturally devoid of light chains as well as single domain antibodies that have a human heavy chain domain have been described (Muyldermans J Biotechnol. 2001 Jun;74(4):277-302; Holliger Nat Biotechnol. 2005 Sep;23(9):1126-362). Antigen binding single V_{H} domains have also been identified from, for example, a library of murine V_{H} genes amplified from genomic DNA from the spleens of immunized mice and expressed in *E. coli* (Ward et al., 1989, Nature 341: 544-546). Ward et al. named the isolated single V_{H} domains "dAbs" for "domain antibodies." The term "dAb" or "sdAb" as used herein generally refers to a single immunoglobulin variable domain (V_{H}, V_{HH} or V_{L}) polypeptide that specifically binds antigen. Such a molecule only has the V_{H} or V_{L} binding domain respectively, but does not comprise other parts of a full length antibody. Unless otherwise specified, as used herein, the term refers to a single domain antibody that has a V_{H} domain. For use in therapy, human single domain antibodies are often preferred, primarily because they are not as likely to provoke an immune response when administered to a patient.

The terms "single domain antibody", "sdAb", "V_{H} domain antibody", "single V_{H} domain antibody", "V_{H} single domain antibody", "single variable domain", "single variable domain antibody", "single variable heavy chain domain antibody" or immunoglobulin single variable domain (ISV)" are thus all well known in the art and describe the single variable fragment of an antibody that binds to a target antigen. These terms are used interchangeably herein.

Antibodies can be engineered to certain formats well known in the art and can, for example be multispecific, e.g. bispecific or trispecific. Other well-known formats are dual affinity retargeting antibodies (DARTs), as well as bi- and tri-specific killer engager antibodies (BiTE, BiKEs and TriKEs) and Tandem diabodies (TandAbs). Such formats are within the scope of the invention.

A binding molecule as used herein comprises an antibody or antigen binding fragment thereof.

The terms "antigen(s)" and "epitope(s)" are well established in the art and refer to the portion of a protein or polypeptide which is specifically recognized by a component of the immune system, e.g. an antibody or a T-cell / B-cell antigen receptor. As used herein, the term "antigen(s)" encompasses antigenic epitopes, e.g. fragments of antigens which are recognized by, and bind to, immune components. Epitopes can be recognized by antibodies in solution, e.g. free from other molecules. Epitopes can also be recognized by T-cell antigen receptors when the epitope is associated with a class I or class II major histocompatibility complex molecule.

The term "antigen" as used herein therefore refers to the protein or polypeptide which is specifically recognised by the antibody or antigen binding fragment thereof according to the invention. The terms antigen and target antigen are used interchangeably herein. By target, e.g. therapeutic target, is therefore meant the protein or polypeptide the antibody or antigen binding fragment thereof according to the invention specifically binds.

The term "epitope" or "antigenic determinant" refers to a site on the surface of an antigen to which an immunoglobulin, antibody or antibody fragment specifically binds. Generally, an antigen has several or many different epitopes and reacts with many different antibodies. The term "specifically" includes linear epitopes and conformational epitopes.

Epitopes within protein antigens can be formed both from contiguous amino acids (usually a linear epitope) or non-contiguous amino acids juxtaposed by tertiary folding of the protein (usually a conformational epitope). Epitopes formed from contiguous amino acids are typically, but not always, retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods for determining what epitopes are bound by a given antibody or antibody fragment (i.e., epitope mapping) are well known in the art and include, for example, immunoblotting and immunoprecipitation assays, wherein overlapping or contiguous peptides are tested for reactivity with a given antibody or antibody fragment. One method is Pepscan, a procedure for mapping and characterizing epitopes involving the synthesis of overlapping peptides and analysis of the peptides in enzyme-linked immunosorbent assays (ELISAs), see Westwood and Hay, Epitope Mapping: A Practical Approach, Oxford University Press, 2001. Competition assays can also be used to determine if a test antibody binds to the same epitope as a reference antibody.

In one embodiment, the epitope for binding antigen is linear or conformational.

The degree of competition can be expressed as a percentage of the reduction in binding. Such competition or indeed binding can be measured using a real time, label-free bio-layer interferometry assay, e.g., on an Octet RED384 biosensor (Pall ForteBio Corp.), ELISA (enzyme-linked immunosorbent assays) or SPR (surface plasmon resonance), HTRF; flow cytometry; fluorescent microvolume assay technology (FMAT) assay, Mirrorball, high content imaging based fluorescent immunoassays, radioligand binding assays, bio-layer interferometry (BLI), surface plasmon resonance (SPR) and thermal shift assays.

Binding of ADAs to the antigen binding region can neutralise the activity of the antibody. Without wishing to be bound by theory, the inventors have found that ADAs bind to residues in the antibody or antigen binding fragment thereof that are located in an epitope for antigen binding or in proximity thereto, e.g. within 10 residues N or C terminally thereof. In one embodiment, an epitope for antigen binding or part thereof is located in a heavy chain and/or light chain CDR1, 2 or 3 regions. Moreover, if the epitope is located within a CDR region, residues within 10 residues N- or C terminally may also be involved in antigen binding and may be altered to introduce glycosylation site(s) as the addition of a glycan at the engineered motif may also interfere with ADA binding.

The term "anti-drug antibody" or "ADA" as used herein denotes an antibody produced by the adaptive immune system of the recipient of an antibody or antigen binding fragment thereof, e.g. a therapeutic antibody or antigen binding fragment thereof against said therapeutic antibody or antigen binding fragment thereof after administration thereof to the recipient. Thus, as used herein, in one embodiment, ADAs according to the invention are formed in response to the administration of the drug (i.e. treatment-emergent ADAs) and the term does not include pre-existing antibodies that bind the antibody or antigen binding fragment thereof. In another embodiment, the term "ADA" includes pre-existing antibodies that bind the antibody or antigen binding fragment thereof. In one embodiment, "ADA" include both, ADAs formed in response to the administration of the drug and pre-existing antibodies.

ADAs bind an antibody or antigen-binding fragment thereof. ADA binding can be measured using routine techniques and examples are provided herein.

The term "immunogenicity" as used herein denotes the potential of a therapeutic antibody or fragment thereof to induce an immune response in humans or animals. Immunogenicity occurs when the immune system perceives "foreignness" in the biological product and launches specific immune responses against it. During drug development, immunogenicity can be assessed by the measurement of non-neutralizing or neutralizing anti-drug antibodies (ADA) that bind specifically to the biologic and neutralise it.

The aim of engineering a variable region (including a CDR) of an antibody as described herein is to reduce the immunogenicity of the antibody or antigen binding fragment thereof. The immunogenic potential of monoclonal antibodies is mainly manifested in the generation of ADAs. The presence of ADAs has significant clinical impact, altering the drug's pharmacokinetic and pharmacodynamic properties, and lowering serum half-life and bioavailability, thereby ultimately reducing drug efficacy and safety. Therefore, it can be seen that engineering certain modifications into antibodies to reduce their immunogenicity, whether an immune response is reduced or consequences following an immune response are reduced, is advantageous, especially for therapeutic antibodies which may otherwise be hindered by ADAs when trying to bind target. An antibody or fragment thereof according to the present invention may reduce an immune response in that ADAs do not form, and/or may block an interaction with any ADAs that do form. In one embodiment, interaction is blocked.

The term "ADA response" as used herein refers to the generation and/or blocking of ADAs, thus leading to immunogenicity which results in adverse impacts on drug safety and efficacy.

As reviewed in Gunn et al, Clin Exp Immunol. 2016 May;184(2):137-46, ADAs include neutralizing antibodies (NAb) that bind to the drug and inhibit its pharmacological function by preventing target binding and non-neutralizing antibodies (non-NAb) that bind to sites on the drug molecule that do not affect target binding and thereby do not impact the drug's pharmacodynamic activity. Non-NAb are often referred to as 'binding antibodies'.

NAbs can inhibit drug activity soon after the drug is administered, but non-NAb do not inhibit the pharmacodynamic activity of the drug; however, the latter can lower the drug's systemic exposure just as well by increasing the rate of drug clearance, resulting in a clinically similar outcome to that of Nabs - reduced clinical efficacy. Both types of ADA can form immune complexes upon binding drug, which are cleared by the reticuloendothelial system leading to complete elimination within days.

Methods for the detection measurement of the ADA response, including ADA binding to antibodies, are well known, for a review see Gunn et al, supra and Suh et al, Sep Sci. 2022 Jun;45(12):2077-2092. These methods include the use of enzyme-linked immunosorbent assay (ELISA), electrochemiluminescence immunoassay (ECLIA) methods, surface plasmon resonance (SPR) and bio-layer interferometry (BLI) and High-pressure liquid chromatography (HPLC)-based methods.

Methods for epitope mapping of ADAs are also well know and include methods mentioned above, including ELISA-based epitope mapping, see also for example Schick et al, MAbs 2022 Jan-Dec;14(1), Stubenrauch et al, Journal of Pharmaceutical and Biomedical Analysis, Volume 114, 10 October 2015, Pages 296-304; Scharnetzki et al, Molecular Genetics and Metabolism Volume 131, Issues 1-2, September-October 2020, Pages 229-234. A skilled person would therefore be able to determine the residues of an antibody or antigen binding fragment thereof that are involved in the binding of ADAs.

In one embodiment, the epitope in the antibody or antigen fragment thereof to which ADAs bind is a linear epitope or a confirmational epitope. In other words, the one or more amino acid residue of the variable region, e.g. CDR region of the antibody or antigen binding fragment thereof is involved in binding of ADAs by forming part of a linear of conformational epitope. Assays that can be used to determine the epitope are described above.

According to the invention, ADA binding of a modified antibody or antigen binding fragment thereof is reduced. Reduction is compared to the unmodified antibody or antigen binding fragment thereof, e.g. the reference sequence. Reduction can be by at least 10%, 20%, 30%, 40%, 70%, 60%, 70%, 80%, 90%. In one embodiment, ADA binding is substantially abolished.

The term "isolated" refers to a moiety that is isolated from its natural environment. For example, the term "isolated" refers to an antibody or antigen binding fragment thereof that is substantially free of other antibodies or antibody fragments. Moreover, an isolated antibody or antigen fragment thereof is substantially free of other cellular material and/or chemicals. Antibodies or antigen binding fragments thereof of the invention are preferably isolated.

There may be one, two, three, four, five, six, seven, eight, nine, ten, eleven, or more than eleven modifications as described herein to the antibody variable region. As the skilled person will appreciate, oligomerisation of the chains into a dimer of heavy chains and a dimer of light chains means that the single mutant may be duplicated across each chain in the dimer. Therefore, a single mutation may be in effect duplicated across each chain. Therefore, an antibody may have two, four, six, eight, twelve, fourteen, sixteen, eighteen, twenty or twenty-two mutations present. In one embodiment, there is one modification in one or more the variable region(s).

In one embodiment of the invention, the variable region of the antibody or antigen binding fragment thereof is a heavy chain variable region or light chain variable region.

In one embodiment, a CDR1, 2 or 3 region or a region within 20, e.g. 1 to 20, e.g.10, e.g. 1, 2, 3, 4, 5, 6, 7, 8, 9 or 10 amino acid residues of a framework region adjacent to a CDR1, 2 or 3 region is modified.

Amino acids within the CDRs can be defined using the Kabat method. CDRs in the heavy chain refer to CDR1 residues H31-H35, CDR2 residues H50-65, CDR3 residues H95-H102. CDRs in the light chain refer to CDR1 residues L24-L34, CDR2 residues L50-L56, CDR3 residues L89-L97. Insertions of residues in any of the CDRs can occur which are named from A-Z. For example, CDR3 of the heavy chain of adalimumab has 4 insertions after residue 100 and are thus named 100A-D, followed by residue 101. In the examples, residues are allocated numbers using the position as counted form the N terminus.

In one embodiment, a CDR1, 2 or 3 region or a region or within 1, 2, 3, 4 or 5 amino acid residues of a framework region adjacent to a CDR1, 2 or 3 region is modified.

In one embodiment, a CDR1, 2 or 3 region is modified.

In one embodiment, the one or more amino acid residue that is substituted to form the N-X-S/T sequence motif is located in a CDR1, 2 or 3 region or within 20, e.g. 10, e.g. 1, 2, 3, 4, 5, 6, 7, 8, 9 or 10 amino acid residues of a framework region adjacent to a CDR1, 2 or 3 region.

Residues within 10, amino acid residues of a framework region adjacent to a CDR1, 2 or 3 region refers to the 10 residues immediately N-terminal or immediately C-terminal of the CDR1, 2 or 3 region with reference to the linear amino acid sequence.

All, some, or one of the variable regions or CDRs of the engineered antibody may be engineered to comprise one or more sequence motifs. The sequence motifs may be engineered into variable regions or CDRs of the heavy chain or light chain.

In one embodiment, the one or more amino acid residue is located in a CDR1, 2 or 3 region or within 1, 2, 3, 4 or 5 amino acid residues of a framework region adjacent to a CDR1, 2 or 3 region.

In one embodiment, the one or more amino acid residue is located in a CDR1, 2 or 3 region.

In a separate aspect, the invention also relates to an antibody or antigen binding fragment thereof comprising a modified amino acid sequence wherein one or more amino acid residue located in a CDR1, 2 or 3 region or within 20, e.g. 10 amino acid residues of a framework region adjacent to a CDR1, 2 or 3 region is substituted with N, T or S to introduce a N-X-S/T sequence motif wherein X is any amino acid other than proline and wherein said amino acid residue is located in an epitope for binding antigen.

As used herein, the term "sequence motif' is given its usual meaning in the art and refers to an amino acid sequence that is usually conserved and related to a discrete biological function. A sequence motif may also be referred to as a "consensus sequence" or a "sequon".

In the context of glycosylation of target proteins, an oligosaccharide may be transferred to selected residues within certain sequence motifs within polypeptide chains. In particular, glycosylation sites are characterized by the N-X-S/T sequon, which acts as an acceptor sequence for N-linked glycosylation. An N-X-S/T sequence motif is a sequence of amino acids having an asparagine residue upstream of an 'X' amino acid, where X can be any amino acid other than proline, followed by a serine or threonine residue.

N-glycosylation is thus a post-translational modification (PTM) attached to asparagine (Asn or N) amino acid residues within the N-(X)-S/T sequon, where X is any amino acid residue except proline, while S is serine and T is threonine. An N-glycan is formed. This PTM in antibodies can take place naturally or be introduced through engineering at both fragment crystallizable region (Fc) and fragment antigen-binding (Fab) variable region. This most frequent and complex PTM plays an important role in antibody functions and properties.

N-glycosylation in the constant Fc region is well characterized in all human antibody isotypes and modulates the Fc-mediated immune response through Fc receptors and complement system protein interaction.

In one embodiment, this sequence motif can be created by engineering into the amino acid sequence of an antibody or fragment thereof an asparagine residue upstream of any suitable X-SΓΓ sequence. However, in another embodiment, it is also possible that the N-X-S/T sequence motif is created by engineering into the amino acid sequence of an antibody or fragment thereof a serine or threonine residue downstream of any suitable N-X sequence. For example, engineering can be through an amino acid substitution of an existing residue with N, S or T or alternatively, a de novo residue may be inserted.

For example, an amino acid wild type residue X1 located in a sequence motif X1XS (wherein X is any amino acid and the two X may or may not be identical amino acids) is replaced with N to yield a NXS motif. Similarly, a wild type residue X1 located in a sequence motif NXX1 (wherein X is any amino acid and the two X may or may not be identical amino acids) is replaced with N to yield a NXS motif.

In one embodiment, the one or more (wild type) amino acid residue is replaced with N. In one embodiment, the one or more (wild type) amino acid residue is replaced with T. In one embodiment, the one or more (wild type) amino acid residue is replaced with S.

In another embodiment, it is also possible to engineer into the sequence an entire N-X-S/T sequence motif, in particular where there is no suitable N or SΓΓ available. For instance, in an amino acid sequence in which there are no suitable N-X or X-SΓΓ sequence, or where, if there is a suitable N-X or X-SΓΓ sequence, this sequence may be hidden within an inaccessible portion of the three-dimensional structure of the protein so that no (or insufficient) co- or post-translational modifications may be made to the N.

Thus, in one embodiment, one wild type residue is replaced to yield the N-X-S/T sequence motif. In another embodiment, two or three wild type residue are replaced to yield the N-X-S/T sequence motif.

The 'X' amino acid is not to be proline, as proline is known in the art to disrupt protein structures for example by breaking alpha helices within the secondary protein structure. Studies have indicated a lack of glycosyl-acceptor capabilities of sequons where 'X' is proline, potentially due to their inability to adopt a turn or loop conformation which permits the interaction between the hydroxy amino acid and the asparagine residue. In some embodiments, 'X' may be a canonical or a non-canonical amino acid. Where 'X' is a canonical amino acid, 'X' may be any of the following amino acids: alanine (A, Ala), arginine (R, Arg), asparagine (N, Asn), aspartate (D, Asp), cysteine (C, Cys), glutamate (E, Glu), glutamine (Q, Gln), (G, Gly), histidine (H, His), isoleucine (I, Ile),leucine (L, Leu), lysine (K, Lys), methionine (M, Met), phenylalanine (F, Phe), proline (P, Pro), serine (S, Ser), threonine (T, Thr), tryptophan (W, Trp), tyrosine (Y, Tyr), or valine (V, Val).

The antibody or antigen-binding fragment thereof according to the present invention may comprise one or more engineered N-X-S and/or N-X-T sequence motifs, for example one, two, three, four, five, six, seven, eight, nine, ten, eleven or more than eleven N-X-S and/or N-X-T sequence motifs, for example between one and five N-X-S and/or N-X-T sequence motifs, for example one or two N-X-S and/or N-X-T sequence motifs.

The N-X-S and/or N-X-T sequence motifs is a motif for glycosylation. Therefore, in one embodiment, the antibody or antigen binding fragment thereof may be engineered to comprise N-linked oligosaccharides on these sequence motifs. An antibody or antigen binding fragment thereof according to the present invention may be modified by the addition of oligosaccharides, such as glycans, to its amino acid sequence. As described herein, glycans may be N-linked in that they are added to nitrogen atoms, for example on asparagine residues within the amino acid sequence of the antibody or antigen-binding fragment thereof. N-glycans may be incorporated onto existing asparagine residues within existing N-X-S and/or N-X-T sequence motifs, or onto N-X-S and/or N-X-T sequence motifs that have been engineered into the amino acid sequence. In one embodiment, one or more N-linked oligosaccharides engineered into a variable region of the antibody or antigen-binding fragment thereof. In another embodiment, one or more N-linked oligosaccharides are engineered into a CDR of the antibody or antigen-binding fragment thereof. Thus, in one embodiment, an amino acid sequence of the antibody or fragment thereof is engineered to comprise one or more glycans in a complementarity-determining region (CDR).

As explained above, amino acid modifications may include, but are not limited to, deletions, substitutions/replacements, and insertions. Deletion mutations involve the loss of an amino acid, resulting in a frameshift or decrease in the length of the amino acid sequence. Insertion mutations involve the addition of an amino acid, resulting in a frameshift or increase in the length of the amino acid sequence. Substitution mutations involve the replacement of one amino acid for another. Substitution mutations can be conservative or non-conservative. Any amino acid may be modified, for example any of alanine (A, Ala), arginine (R, Arg), asparagine (N, Asn), aspartate (D, Asp), cysteine (C, Cys), glutamate (E, Glu), glutamine (Q, Gln), (G, Gly), histidine (H, His), isoleucine (I, Ile),leucine (L, Leu), lysine (K, Lys), methionine (M, Met), phenylalanine (F, Phe), proline (P, Pro), serine (S, Ser), threonine (T, Thr), tryptophan (W, Trp), tyrosine (Y, Tyr), and valine (V, Val). According to the present invention, any suitable amino acid may be substituted for an asparagine residue, a serine residue or a threonine residue. Several methods are well known in the art for inducing amino acid modifications. As will be readily understood by the skilled person, a nucleic acid molecule encoding the modified amino acid sequence of interest may be generated by several means (i.e., de novo synthesis) which are known in the art.

Any such amino acid modification may be present in the antibody or antigen binding fragment thereof according to the invention. The modification may involve the addition of one or more glycans to the amino acid sequence of the antibody or antigen binding fragment thereof. As used herein, the term "glycan" may refer to sugar-containing compounds linked so as to form a chain. The term may also refer to sialic acids and GIcNAcs. By "sugar" we intend units such as glucose, mannose, fucose and fructose. Examples of glycans include, but are not limited to, oligosaccharides, polysaccharides, polyethylene glycols (PEG), and polypropylene glycols (PPG). Oligosaccharides may be, but are not limited to, glycans such as N-glycans, O-glycans, and C-glycans. It is a preference of the present invention for the amino acid sequence to be modified by the addition of N-linked oligosaccharides, for example glycans, in particular N-glycans. The addition of glycans to an amino acid sequence is known as glycosylation.

As used herein, the term "glycosylation" refers to the process by which oligosaccharides (glycan moieties) are attached to a target molecule, such as proteins and lipids. The skilled person will readily recognise that this process is vital for both the functioning and stability of the protein. Oligosaccharides are carbohydrates consisting of chains or polymers of monosaccharides (single sugar molecules). Glycosylation is a form of co-translational and post-translational modification of proteins in which carbohydrates are conjugated or covalently attached onto a protein. In biology, the process of glycosylation is an enzyme-catalysed reaction, and may involve enzymes such as oligosaccharyltransferase (OSTs) and glycosyltransferases. Within mammalian cells, glycosylation primarily occurs within the rough endoplasmic reticulum, cytoplasm, and nucleus. There are two major types of glycosylation: N-glycosylation (also known as "N-linked glycosylation") and O-glycosylation (also known as "O-linked glycosylation"), with the former being the most prevalent. There is also C-glycosylation (also known as "C-linked glycosylation") which occurs when a glycan is linked to a carbon atom on a tryptophan side chain. In N-glycosylation, glycans are attached to the nitrogen atoms of amino acids (for example, asparagine and arginine). In O-glycosylation, glycans are attached to the oxygen atoms of a hydroxyl group on amino acids (for examine, serine, threonine, and tyrosine). Accordingly, in the context of the present invention, the addition of "N-glycans" to an amino acid sequence of the antibody or antigen-binding fragment thereof is of particular interest.

In one embodiment, there may be one or more glycans glycanengineered into a CDR of the antibody or antigen-binding fragment thereof. Where there are two or more than two glycans, the glycans may be of the same type or of different types. There may be one, two, three, four, five, six, seven, eight, nine, ten, eleven, or more than eleven glycans engineered into a CDR of a single chain. As described herein, the skilled person will appreciate that, once expressed, the chains oligomerise such that a dimer of heavy chains and a dimer of light chains is present in a single antibody. Therefore, a single glycan may be in effect duplicated across each chain. Therefore, an antibody may have two, four, six, eight, twelve, fourteen, sixteen, eighteen, twenty or twenty-two glycans present in a CDR. However, the preference is that there may be one glycans engineered into a CDR (i.e., one per chain monomer in a dimer such that the glycan is in effect duplicated). One or more CDRs may be engineered to comprise one or more glycans. All, some, or one of the CDRs of the engineered antibody may be engineered to comprise one or more glycans. The glycans may be engineered into CDRs of the heavy chain and/or light chain. For example, one or more glycans may be present on a heavy chain. One or more glycans may be present on a light chain. One or more glycans may be present on a heavy chain and a light chain.

In accordance with another of the invention, an amino acid sequence of the antibody or antigen-binding fragment thereof is engineered to reduce immunogenicity of the antibody or fragment thereof when the antibody or fragment thereof is administered to a subject, wherein the amino acid sequence is engineered to comprise one or more N-linked oligosaccharides in a variable region of the monoclonal antibody or antigen-binding fragment thereof. In one embodiment, the N-linked oligosaccharides may be N-glycans. In one embodiment, the monoclonal antibody or antigen-binding fragment thereof is engineered to comprise one or more N-linked oligosaccharides in a complementarity-determining region (CDR) of the monoclonal antibody or antigen-binding fragment thereof or antibody. Again, there may be one, two, three, four, five, six, seven, eight, nine, ten, eleven, or more than eleven N-linked oligosaccharides engineered into the antibody variable region(s). For example, there may be one or two N-linked oligosaccharides engineered into the variable region(s), more for example into the CDR. One or more CDRs may be engineered to comprise one or more N-linked oligosaccharides. All, some, or one of the CDRs of the antibody may be engineered to comprise one or more N-linked oligosaccharides. The N-linked oligosaccharides may be engineered into CDRs of the heavy chain and/or light chain. For example, one or more N-linked oligosaccharides may be present on a heavy chain. One or more N-linked oligosaccharides may be present on a light chain. One or more N-linked oligosaccharides may be present on a heavy chain and a light chain.

In one embodiment, the antibody or antigen-binding fragment thereof is engineered to comprise one or more N-glycans in a complementarity-determining region (CDR) of the monoclonal antibody or antigen-binding fragment thereof or antibody. In this aspect, the N-linked oligosaccharides may be incorporated onto existing asparagine residues within the antibody sequence, or those which have been engineered into the antibody sequence as described herein. The possible amino acids to be mutated and the combinations thereof on to which N-linked oligosaccharides may be incorporated may be as described herein.

For example, one, two, three, four, five, six, seven, eight, nine, ten, eleven or more than eleven N-linked oligosaccharides may be included. The N-linked oligosaccharides may be incorporated onto asparagine residues which are naturally present in the antibody sequence, or which have been engineered into the antibody sequence. In one embodiment, the N-linked oligosaccharides may be N-glycans.

The advantage of using oligosaccharides such as N-glycans as the preferred modification is that these moieties are naturally occurring and therefore represent an attractive option for reducing immunogenicity and immune reactivity of antibodies. A further benefit is that N-glycans can be easily added to amino acid sequences, for example in a cell which expresses (or is engineered to express) the amino acid sequences. This means that no additional post-expression, purification or further modification steps are required. Additionally, due to the array of N-glycans, the particular type (i.e., shape, modifications, size) and number N-glycans can be adapted in accordance with the desired function.

For example, whether the N-glycan is capped or afucosylated can impact the activity of the amino acid sequence to which it is attached, as described below.

The skilled person will appreciate that any suitable type of oligosaccharides may be incorporated onto the amino acid sequence of the antibody or fragment thereof. Oligosaccharides are chains of monosaccharide units. An oligosaccharide may be a trisaccharide, for example nigerotriose, maltotriose, melezitose, maltotriulose, raffinose, and kestose. An oligosaccharide may be a tetrasaccharide, for example nigerotetraose, maltotetraose, lychnose, nystose, sesamose, and stachyose. An oligosaccharide may be a pentasaccharide, a hexasaccharide (for example α-Cyclodextrin), a heptasaccharide, an octasaccharide, a nonasaccharide, a decasaccharide and so on. The core structures of N-linked oligosaccharides are often, but not always, pentasaccharides.

N-glycans can be classified into three types. Firstly, oligomannose N-glycans, in which only mannose residues are present. Secondly, complex N-glycans, in which "antennae" are initiated by GIcNAc, extend the core. Thirdly, hybrid N-glycans, in which mannose extends the Manα1-6 arm of the core and one or two GlcNAc-initiated antennae extend the Manα1-3 arm. The preference of the present invention is for the N-glycans to be complex N-glycans.

In one embodiment, the complex N-glycan may be bi-antennary, in that it comprises two antennas. In another embodiment, the complex N-glycan may be tri-antennary, in that it comprises three antennas. In another embodiment, the complex N-glycan may be tetra-antennary, in that it comprises four antennas.

N-glycans may or may not be afucosylated. Afucosylation is the process by which fucose residues are removed from carbohydrate chains. Therefore, in one embodiment, the complex N-glycan may be afucosylated. In another embodiment the complex N-glycan may not be afucosylated. Afucosylation may be achieved via expressing the glycosylated proteins in a cell with a *Fut8* gene knock-out. This knock-out removes expression of the sole enzyme that is responsible for core fucosylation. The advantage of removing core fucosylation stems is that afucosylation is known in the art to optimise the amino acid sequence to which the N-glycan is added. Afucosylation has been found to generate significant increases in antibody-dependent cellular cytotoxicity (ADCC) activity of antibodies N-glycosylated on the Fc fragment (Shields et al, 2002). N-glycans may be capped, for example with sialic acids and/or galactose residues. In one embodiment, the N-glycans are capped with sialic acid. The type of capping on the terminal residue can greatly impact function. Galactose-capped terminal residues can experience enhanced ADCC as described above and complement activation (Raju 2008). Sialic acid-capped residues may result in anti-inflammatory properties. Further, sialic acid-capped residues reduce uptake by the ASGPR receptor. The ASGPR receptor binds to exposed galactose residues and increases clearance of the therapeutic. Therefore, by covering the exposed galactose residues with sialic acid caps, ASGPR-mediated clearance can be reduced (Anthony et al, 2009; Wang, 2019, p.63-75). The skilled person will appreciate that there are several different types of sialic acids that may or may not be present as a cap. In one embodiment, the complex N-glycan may be capped. In another embodiment, the complex N-glycan may not be capped. In some embodiments, the complex N-glycan may be capped with α2,6-linked sialic acid residues. The advantage of the α2,6-linker is that this represents a humanised version of the sialic acid cap. For instance, in CHO cells, sialic acids are linked with an α2,3 linker, however the α2,6 is known to be less immunogenic in humans. Therefore, the addition of the α2,6-linked sialic acid cap to N-glycans added onto the amino acid sequences described herein has the additional effect of further reducing or blocking immunogenicity and therefore reducing hindrances to or enhancing the therapeutic effectiveness of the drug.

In any aspect of the invention, in one embodiment, the antibody or antigen-binding fragment thereof or antibody may comprise afucosylated bi-antennary complex N-glycans capped with a2,6-sialic acids. In another embodiment, the monoclonal antibody or antigen-binding fragment thereof or antibody may comprise afucosylated tri-antennary complex N-glycans capped with a2,6-sialic acids. In another embodiment, the monoclonal antibody or antigen-binding fragment thereof or antibody may comprise afucosylated tetra-antennary complex N-glycans capped with a2,6-sialic acids.

Any antibody of the present invention may be modified so as to comprise any form of the N-glycans described herein. The N-glycan profile or N-glycosylation profile may be homogenous in that all of the N-glycans added to the amino acid sequence are of the same type. The N-glycan profile may alternatively be heterogeneous in that there are varying degrees of each glycan at each site in a population of a protein produced. As used herein, the term "N-glycosylation profile" refers to the site of N-glycosylation and the type of N-glycan present at the site on the surface of the fully folded protein. The N-glycosylation profile therefore reflects both the degree of N-glycosylation site occupancy and the type of individual N-glycans on a fully folded protein. As would be appreciated by the skilled person, in determining the N-glycosylation profile, the degree of N-glycosylation site occupancy and type of N-glycans present will reflect the profile of all proteins in a given population of proteins (i.e., those proteins present in a sample). In particular, in the context of the present invention, the term "N-glycosylation profile" refers to the global profile of the degree of N-glycosylation site occupancy and type of N-glycans present in a given population of recombinant proteins. For example, in the context of the present invention, an improved glycosylation profile consists of an increase in the degree of N-glycosylation site occupancy on the recombinant protein with consistent N-glycan moieties that are reproducible between production batches of recombinant protein. Mass spectroscopy can be utilised in order to determine the % N-glycosylation occupancy of a given protein.

The skilled person will understand that the principle of engineering such modifications into the variable region or CDR of an antibody or antigen binding fragment thereof to reduce the effect of ADAs will apply to any antibody in accordance with any aspect of the invention. Preferably, the antibody or antigen binding fragment thereof is a therapeutic antibody or antigen binding fragment thereof. The antibody or antigen binding fragment thereof may bind to any therapeutic target of interest, for example a target in the therapy of diseases. A skilled person would be familiar with such targets which are, for example reviewed, in Sharma et al, Molecules. 2023 Sep 5;28(18):6438.

Examples of target antigens include, but are not limited to: a transmembrane molecule; a receptor; a ligand; a growth factor; a growth hormone; a clotting factor; an anti-clotting factor; a plasminogen activator; a serum albumin; a receptor for a hormone or a growth factor; a neurotrophic factor; a nerve growth factor; a fibroblast growth factor; an interferon; a colony stimulating factor (CSF); an interleukin (IL); a T-cell receptor; a T-cell co-stimulatory receptor, such as CD137; a surface membrane protein; a viral protein; a tumor associated antigen; an integrin or an interleukin; VEGF; a renin; a human growth hormone; a growth hormone releasing factor; parathyroid hormone; thyroid stimulating hormone; a lipoprotein; alpha-1 -antitrypsin; insulin A-chain; insulin B-chain; proinsulin; follicle stimulating hormone; calcitonin; luteinizing hormone; glucagon; clotting factor VIIIC; clotting factor IX; tissue factor (TP); von Willebrand's factor; Protein C; atrial natriuretic factor; a lung surfactant; urokinase; human urine; tissue-type plasminogen activator (t-PA); bombesin; thrombin; hemopoietic growth factor; tumor necrosis factor-alpha or -beta; enkephalinase; RANTES (Regulated on Activation Normally T-cell Expressed and Secreted); human macrophage inflammatory protein (MIP-I)-alpha; Muellerian-inhibiti ng substance; relaxin A-chain; relaxin B-chain; prorelaxin; mouse gonadotropin-associated peptide; a microbial protein, beta-lactamase; DNase; IgE; a cytotoxic T-lymphocyte associated antigen (CTLA); CTLA-4; inhibin; activin; vascular endothelial growth factor (VEGF); protein A or D; a rheumatoid factor; bone-derived neurotrophic factor (BDNF); neurotrophin-3, -4, -5, or -6 (NT-3, NT-4, NT-5, or NT-6); NGF- beta; platelet-derived growth factor (PDGF); aFGF; bFGF; epidermal growth factor (EGF); insulin-like growth factor-I or -II (IGF-I or IGF-II); des(I-3)-IGF-I (brain IGF-I), an insulin-like growth factor binding protein, erythropoietin; an osteoinductive factor; an immunotoxin; a bone morphogenetic protein (BMP); interferon-alpha, -beta, or -gamma; M- CSF, GM-CSF or G-CSF; IL-1 to IL-10; CD3, CD4, CD8, CD11a, CD11b, CD11c, CD18, CD19, CD20, CD34, CD40, 7 or CD46, an ICAM, VLA-4 or VCAM; or HER2, HER3 or HER4 receptor; a member of the ErbB receptor family; an EGF receptor; HER2, HER3 or HER4 receptor; a cell adhesion molecule; LFA-1 , Mac1, pl50.95, VLA-4, ICAM-1, VCAM, alpha4/beta7 integrin or alphav/beta3 integrin; an alpha or beta subunit of a cell adhesion molecule; antibodies); a growth factor, VEGF; tissue factor (TF); alpha interferon (alpha-IFN); IL-8; IgE; blood group antigens Apo2; PD-1; PD-L1 ; PDL-1, LAG3, TIGIT, OX40, TIM-3, flk2/flt3 receptor; LIV-1, mesothelin, nectin-4, CTLA4, FOLR1, PSMA or TNF-alpha. In one embodiment, the target is not TNF-alpha.

In particular, suitable targets in cancer therapy include checkpoint inhibitors such as PD-1, PDL-1, PDL-2, and CTLA-4. Examples of checkpoint inhibitors include pembrolizumab (Keytruda), ipilimumab (Yervoy), nivolumab (Opdivo) and atezolizumab (Tecentriq). Other targets cancer therapy include immunooncology targets and T cell engagers. Examples are TIGIT, CD40, Mesothelin, OX40 and LAG-3.

Examples of tumor associated antigen targets include, but are not limited to: ADRB3, AFP, ALK, BCMA, beta human chorionic gonadotropin, CA-125 (MLIC16), CAIX, CD123, CD133, CD135, CD135 (FLT3), CD138, CD171, CD19, CD20, CD22, CD24, CD276, CD33, CD33, CD38, CD44v6, CD79b, CD97, CDH3 (cadherin 3), CEA, CEACAM6, CLDN6, CLEC12A (CLL1), CSPG4, CYP1B1, EGFR, EGFRvlll, EpCAM, EPHA2, Ephrin B2, ERBBs (e. g. ERBB2), FAP, FGFR1, folate receptor alpha, folate receptor beta, Fos-related antigen, GA733, GD2, GD3, GFRalpha4, globoH, GPC3, GPR20, GPRC5D, HAVCR1, Her2/neu (HER2), HLA-A2, HMWMAA, HPV E6 or E7, human telomerase reverse transcriptase, IL-11 Ra, IL-13Ra2, intestinal carboxyl esterase, KIT, Legumain, LewisY, LMP2, Ly6k, MAD-CT-1, MAD-CT-2, ML-IAP, MN-CA IX, MSLN, MUC1, mut hsp 70-2, NA- 17, NCAM, neutrophil elastase, NY-BR-1, NY-ESO-1, o-acetyl-GD2, OR51E2, PANX3, PDGFR-beta, PLAC1, Polysialic acid, PSCA, PSMA, RAGE1, ROR1, sperm protein 17, SSEA-4, SSTR2, TAG72, TARP, TEM1/CD248, TEM7R, thyroglobulin, Tn antigen, Tn-O-Glycopeptides, TPBG (5T4), TRP-2, TSHR, LIPK2 and VEGFR2. Examples are: CD138, CD79b, TPBG (5T4), HER2, MSLN, MUC1 , CA-125 (MUC16), PSMA, BCMA, CD19, EpCAM, CLEC12A (CLL1), CD20, CD22, CEA, CD33, EGFR, GPC3, CD123, CD38, CD33, CD276, CDH3 (cadherin 3), FGFR1, SSTR2, CD133, EPHA2, HLA-A2, IL13RA2, ROR1 , CEACAM6, CD135, GD-2, GA733, CD135 (FLT3), CSPG4 and TAG-72. Particular examples are: CD138, CD79b, CD123, MSLN, PSMA, BCMA, CD19, CD20, CEA, CD38, CD33, CLEC12a, HER2 and ROR1.

The antibody may be targeted against a target useful in medical therapy of a disease. Such diseases are listed below.

Examples of antibodies according to the invention which are engineered/modified to reduce immunogenicity include, but are not limited to those listed in Lyo et al Antib Ther. 2022 Oct; 5(4): 233-257.

In one embodiment, examples of antibodies according to the invention which are engineered/modified to reduce immunogenicity include but are not limited to Muromonab, Nebacumab, Abciximab, Edrecolomab, Daclizumab, Rituximab, Infliximab, Trastuzumab, Palivizumab, Basiliximab, Gemtuzumab ozogamicin, Alemtuzumab, Adalimumab, Ibritumomab tiuxetan, Omalizumab, Efalizumab, Alefacept, Iodine 131 tositumomab, Cetuximab, Bevacizumab, Natalizumab, Tocilizumab, Abatacept, Panitumumab, Iodine 131 derlotuximab biotin, Iodine 131 metuximab, Nimotuzumab, Ranibizumab, Eculizumab, Racotumomab, Rilonacept, Certolizumab pegol, Romiplostim, Canakinumab, Catumaxomab, Ustekinumab, Ofatumumab, Golimumab, Denosumab, Brentuximab vedotin, Belimumab, Ipilimumab, Pertuzumab, Raxibacumab, Mogamulizumab, Trastuzumab emtansine, Obinutuzumab, Conbercept, Itolizumab, Blinatumomab, Pembrolizumab, Vedolizumab, Secukinumab, Ramucirumab, Efmoroctocog alfa, Siltuximab, Nivolumab, Elotuzumab,Alirocumab, Mepolizumab, Necitumumab, Idarucizumab, Dinutuximab, Daratumumab, Evolocumab, Atezolizumab, Olaratumab, Bezlotoxumab, Brodalumab, Ixekizumab, Reslizumab, Obiltoxaximab, Inotuzumab ozogamicin, Sarilumab, Dupilumab, Durvalumab, Avelumab, Emicizumab, Benralizumab, Ocrelizumab, Guselkumab, Erenumab, Moxetumomab pasudotox, Ravulizumab, Fremanezumab, Sintilimab, Ibalizumab, Galcanezumab, Tildrakizumab, Emapalumab, Cemiplimab, Toripalimab, Caplacizumab, Lanadelumab, Burosumab, Tislelizumab, Risankizumab, Trastuzumab deruxtecan, Brolucizumab, Crizanlizumab, Enfortumab vedotin, Romosozumab, Efgartigimod alfa, Camrelizumab, Polatuzumab vedotin, Luspatercept, Rabimabs, Netakimab, Inebilizumab, Teprotumumab, Sacituzumab govitecan, Atoltivimab, Odesivimab, Maftivimab, Cetuximab sarotalocan, Isatuximab, Belantamab mafodotin, Levilimab, Margetuximab, Satralizumab, Tafasitamab, Naxitamab, Ansuvimab, Eptinezumab, Prolgolimab, Olokizumab, Penpulimab, Dostarlimab, Evinacumab, Sugemalimab, Envafolimab, Regdanvimab, Amubarvimab, Romlusevimab, Amivantamab, Zimberelimab, Bimekizumab, Loncastuximab tesirine, Tisotumab vedotin, Tralokinumab, Tezepelumab, Aducanumab, Disitamab vedotin, Sotrovimab, Anifrolumab, Ormutivimab, Faricimab, Sutimlimab, Mosunetuzumab, Cadonilimab, Nemolizumab, Serplulimab.

In some aspects, the antibody is an immune checkpoint inhibitor. In some embodiments, the antibody targets cytotoxic T- lymphocyte antigen-4 (CTLA-4). In some aspects, the immune checkpoint inhibitor that targets CTLA-4 is Ipilimumab or tremelimumab (ticilimumab, CP-675.206). In some aspects, the immune checkpoint inhibitor targets PD-1. In some aspects, the immune checkpoint inhibitor that targets PD-1 is nivolumab (ONO-4538/BMS-936558, MDX1106, OPDIVO^{®}), pembrolizumab (MK-3475, KEYTRUDA^{®}), pidilizumab (CT-011), atezolizumab (MPDL328OA), cemiplimab (LIBTAYO^{™}), Spartalizumab (PDR001), Camrelizumab (SHR1210), Sintilimab (IBI308), Tislelizumab (BGB-A317), Toripalimab (JS 001), AMP-224, AMP-514 or Spartalizumab (PDR001). In some aspects, the immune checkpoint inhibitor targets PD-L-1 In some aspects, the immune checkpoint inhibitor that targets PD-L-1 is Avelumab, Atezolizumab, Durvalumab, KN035, CK-301, AUNP12, CA- 170, or BMS-986189. In some aspects, the immune checkpoint inhibitor targets T cell immunoglobulin and mucin-domain containing-3 (TIM-3) and/or Lymphocyte Activating 3 (LAG3) proteins. In some aspects, the immune checkpoint inhibitor targeting TIM3 is MBG453; TSR-022; or LY3321367. In some aspects, the immune checkpoint inhibitor targeting LAG3 is IMP321 (Eftilagimod alpha), BMS-986016 (Relatlimab), LAG525 (anti- LAG-3 mAb), REGN3767 (anti-LAG-3 mAb), TSR-033 (anti-LAG-3 mAb). MGD013 (a PD-I/LAG-3 bispecific DART^{®} protein), or FS118 (a LAG-3/PD-L1 bispecific antibody).

### Nucleic acids, vectors, hosts

In another aspect, the invention relates to a nucleic acid molecule, e.g. an isolated nucleic acid molecule, comprising a nucleic acid encoding an antibody or antigen binding fragment thereof as defined above.

The term "nucleic acid," "polynucleotide," or "nucleic acid molecule" refers to deoxyribonucleic acid (DNA) or ribonucleic acid (RNA), or a combination of a DNA or RNA. RNA includes *in vitro* transcribed RNA, synthetic RNA or mRNA sequence. The nucleic acid construct may further comprise a suicide gene. The construct may be in the form of a plasmid, vector, transcription or expression cassette.

In another aspect, the invention relates to an isolated nucleic acid construct comprising a nucleic acid as defined above. The construct may be in the form of a plasmid, vector, transcription or expression cassette.

The nucleic acid can be cloned into a number of types of vectors. For example, the nucleic acid can be cloned into a vector including, but not limited to a plasmid, a phagemid, a phage derivative and a cosmid. Vectors of particular interest include expression vectors, replication vectors, probe generation vectors, and sequencing vectors. The vectors can be suitable for replication and integration eukaryotes. Typical cloning vectors contain transcription and translation terminators, initiation sequences, and promoters useful for regulation of the expression of the desired nucleic acid sequence.

In one embodiment, the vector is an *in vitro* transcribed vector, e.g., a vector that transcribes RNA of a nucleic acid molecule described herein. The expression vector may be provided to a cell in the form of a viral vector. Viral vector technology is well known in the art and is described, for example, in Sambrook et al. (Molecular Cloning: A Laboratory Manual, Cold Spring Harbor Laboratory, New York, 2013). A number of viral based systems have been developed for gene transfer into mammalian cells. For example, retroviruses such as adenovirus vectors can be used. In one embodiment, a lentivirus vector is used.

To produce the antibodies, monoclonal antibodies or antigen-binding fragments thereof, the aforementioned expression vectors are used for stable transfection or transient transfection of a host cell, such as a mammalian cell. A stable transfection refers to any method in which genes are integrated into the host genome of a cell that allows for stable expression of the genes under the control of a constitutive or inducible promoter. A stable transfection allows for integration into the host genome thus the gene is replicated, and the expression of the genes are sustained long-term. A transient transfection refers to any method in which genes are introduced and expressed under the control of a constitutive or inducible promoter. A transient transfection does not allow for integration into the host genome thus the gene is not inherited during cell division, and the expression of the genes is therefore for a finite period of time. The expression vector may be a plasmid.

In a further aspect, the invention also relates to an isolated cell or cell population comprising one or more nucleic acid construct or vector as described above. In one embodiment, the cell is an isolated recombinant host cell comprising one or more nucleic acid construct as described above. The host cell may be a bacterial, viral, plant, mammalian or other suitable host cell.

Such host cells are well known in the art and many are available from the American Type Culture Collection (ATCC). These host cells include, inter alia, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, HEK-293 cells and a number of other cell lines. Mammalian host cells include human, mouse, rat, dog, monkey, pig, goat, bovine, horse and hamster cells. Other cell lines that may be used are insect cell lines (e.g., Spodoptera frugiperda or Trichoplusia ni), amphibian cells, bacterial cells, such as E. coli, plant cells and fungal cells. Fungal cells include yeast and filamentous fungus cells including, for example, Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella. Derivatives of known cells lines, e.g. genetically modified cell lines can also be used.

In one embodiment, a method of making the antibody or antigen binding fragment thereof as described herein is provided, wherein the method comprises culturing the host cell under conditions suitable for expression of the polynucleotide encoding an antibody or antigen binding fragment thereof as described herein and isolating the antibody or antigen binding fragment thereof.

### Exemplary modifications

In one embodiment, an antibody or antigen binding fragment thereof according to the invention comprises a further moiety, e.g. is conjugated/linked to another moiety. The further moiety may a therapeutic, cytotoxic moiety or other moiety. The therapeutic moiety may be an antibody molecule or fragment thereof (e.g., a Fab, F(ab')2, Fv, a single chain Fv fragment (scFv) or a single domain antibody, for example a V_{H} domain, antibody mimetic protein which binds to a target of interest.

Thus, another aspect, the invention also relates to a binding molecule comprising an antibody or antigen binding fragment thereof as described herein. Such a binding molecule may, for example, include a second antibody or antigen binding fragment thereof, binding to the same or different target.

In another embodiment, the further moiety is a tag/label, for example to visualise / detect the antibody or antigen binding fragment thereof.

In another embodiment, the further moiety may serve to prolong the half-life of the antibody or antigen binding fragment thereof. The further moiety may comprise a protein, for example a peptide, antibody, or part thereof, such as a V_{H} domain or CDR, that binds a serum albumin, e.g., human serum albumin (HSA) or mouse serum albumin (MSA). In one embodiment, the further moiety may comprise a V_{H} domain that binds serum albumin, e.g., human serum albumin (HSA) or mouse serum albumin (MSA). The further moiety may comprise a serum albumin, e.g. a HSA or a variant thereof such as HSA C34S. PEGylation or incorporation in a liposome or may also be used to increase half-life.

The term "half-life" as used herein refers to the time taken for the serum concentration of the antibody to be reduced by 50%, in vivo, for example due to degradation of the sequence or compound and/or clearance or sequestration of the sequence or compound by natural mechanisms. Half-life may be increased by at least 1.5 times, preferably at least 2 times, such as at least 5 times, for example at least 10 times or more than 20 times. For example, increased half-life may be more than 1 hours, preferably more than 2 hours, more preferably more than 6 hours, such as more than 12 hours, or even more than 24, 48 or 72 hours. The in vivo half-life of an antibody of the invention can be determined in any manner known per se, such as by pharmacokinetic analysis. Suitable techniques will be clear to the person skilled in the art. Half-life can for example be expressed using parameters such as the t1/2-alpha t1/2-beta and the area under the curve (AUC).

In one embodiment, the antibody or antigen binding fragment thereof is labelled with a detectable or functional label. A label can be any molecule that produces or can be induced to produce a signal, including but not limited to fluorophores, fluorescers, radiolabels, enzymes, chemiluminescers, a nuclear magnetic resonance active label or photosensitizers. Thus, the binding may be detected and/or measured by detecting fluorescence or luminescence, radioactivity, enzyme activity or light absorbance.

In still other embodiments, the antibody or antigen binding fragment thereof is coupled to at least one therapeutic moiety, such as a drug, an enzyme or a toxin. In one embodiment, the therapeutic moiety is a toxin, for example a cytotoxic radionuclide, chemical toxin or protein toxin. For example, the antibody or antigen binding fragment thereof is attached to a "payload" and forms part of an Antibody Drug Conjugate (ADC).

There may be one or more payloads (e.g., a dual payload). Examples of a payload may include, but are not limited to, antibodies or fragments thereof, T-cell receptors, chemokines, interleukins (e.g., IL-2, IL-3, IL-4, IL-5, IL-6, IL-7, IL-8, IL-9, IL-10, IL-11, IL-12, IL-13, IL-14, IL-15, IL-16, IL-17, IL-18, IL-21) or chemokines (e.g. CXCL9, CXCL10, CXCL11, CCL5, CCL2, CX3CL1), cytotoxic agents, cytostatic agents, inhibitors (e.g., DNA replication inhibitors, tubulin inhibitors, RNA inhibitors), DNA damaging agents, antibiotic agents, antiviral agents, immunotherapeutic agents and immunopayloads, chemotherapeutic agents and other anti-cancer agents, anti-inflammatory agents, other therapeutic agents, any other compounds suitable for use in an ADC, and dual payloads. The payload may also be a tissue-targeting sequence or a signal sequence. The advantage of ADCs combines the specificity of the antibody to its target and the therapeutic effect of the payload, to target the payload to the target site of the antibody. By "attachment" we intend any form of fusion, conjugation, or linkage, which may be covalent or non-covalent, cleavable or non-cleavable, and peptide or chemical.

Linkage to another moiety may be using a peptide linker, e.g. a G4S linker, by cysteine conjugation or other methods available in the art.

### Methods for making an antibody or antigen binding fragment thereof and methods for reducing immunogenicity

The invention also relates to methods for producing an antibody or antigen binding fragment thereof as described above, producing an antibody or antigen binding fragment thereof with reduced immunogenicity and methods for reducing the ADA response of an antibody or antigen binding fragment thereof.

In one aspect, the invention relates to a method for producing an antibody or antigen binding fragment thereof with reduced immunogenicity comprising modifying the amino acid sequence of the variable region to comprise one or more N-X-S and/or N-X-T sequence motifs wherein X is any amino acid other than proline.

In one embodiment, said one or more motif is located in an epitope for binding antigen or in proximity thereto.

In one embodiment, one or more amino acid residue located in a variable region is substituted with N, T or S to introduce a N-X-S/T sequence motif and wherein said amino acid residue is located in an epitope for binding antigen or in proximity thereto.

In one embodiment, a N-X-S/T sequence motif is inserted into an amino acid residue sequence located in an epitope for binding antigen or in proximity thereto.

In one embodiment, the method comprises the steps of
a) substituting one or more amino acid residue in a variable region with a N, S or T to introduce a N-X-S/T sequence motif wherein X is any amino acid other than proline and wherein said one or more amino acid residue is located in an epitope for binding antigen and
b) expressing a nucleotide sequence or nucleic acid encoding the modified antibody or antigen binding fragment thereof in a suitable host cell or host organism.

The invention further relates to a method for reducing the ADA response of an antibody or antigen binding fragment thereof with reduced immunogenicity comprising modifying the amino acid sequence of the variable region to comprise one or more N-X-S and/or N-X-T sequence motifs wherein X is any amino acid other than proline.

In one embodiment, said one or more motif is located in an epitope for binding antigen or in proximity thereto.

In one embodiment, the one or more amino acid residue located in a variable region is substituted with N, T or S to introduce a N-X-S/T sequence motif and wherein said amino acid residue is located in an epitope for binding antigen or in proximity thereto.

In one embodiment, a N-X-S/T sequence motif is inserted into an amino acid residue sequence located in an epitope for binding antigens or in proximity thereto.

In one embodiment, the method comprises the step of determining residues involved in binding to the target antigen. Suitable methods are known in the art and described elsewhere herein. In one embodiment, the method comprises the step of identifying residues within 20, e.g. 10 amino acid residues N terminally adjacent to residues involved in binding to the target antigen and within 20, e.g. 10 amino acid residues C terminally adjacent to residues involved in binding to the target antigen. The method may also involve the further step of mutating one or more, e.g. each of these residues to introduce the N-glycosylation sequon.

In one embodiment, the method comprises the steps of
a) substituting one or more amino acid residue in a variable region with a N, S or T to introduce a N-X-S/T sequence motif wherein X is any amino acid other than proline and wherein said one or more amino acid residue is located in an epitope for binding antigen and
b) expressing a nucleotide sequence or nucleic acid encoding the modified antibody or antigen binding fragment thereof in a suitable host cell or host organism.

In respect of the methods of the various aspects above, in one embodiment, In the method comprises the step of assessing binding of the antibody or antigen binding fragment thereof to its target and/or assessing binding to ADAs.

In one embodiment, the step of assessing binding of the antibody or antigen binding fragment thereof to its target is carried out prior to modification of the sequence and after modification of the sequence.

In one embodiment, the step of assessing binding of the antibody or antigen binding fragment thereof to ADAs is carried out prior to modification of the sequence and/or after modification of the sequence.

In one embodiment, the method comprises the step of determining which residues in the variable region are located in epitopes for ADA binding. This step is carried out prior to modification of the sequence.

Any combination of steps is also covered.

In one particular aspect, the invention provides a method for producing an antibody or antigen fragment thereof / reducing the immunogenicity of an antibody or antigen fragment thereof comprising the steps of
a) Determining one or more residue that bind to target antigen and
b) Determining residues within 20, e.g. 10 amino acid residues N terminally adjacent to one or more residue involved in binding to the target antigen and within 20, e.g. 10 amino acid residues C terminally adjacent to one or more residue involved in binding to the target antigen.
   a) substituting one or more of the residues identified with a N, S or T to introduce a N-X-S/T sequence motif wherein X is any amino acid other than proline;
   b) expressing a nucleotide sequence or nucleic acid encoding the modified antibody or antigen binding fragment thereof in a suitable host cell or host organism;
   c) determining the binding of ADAs to the modified antibody or antigen binding fragment thereof and determining the binding of the modified antibody or antigen binding fragment thereof to the antigen target and
   d) identifying a modified antibody or antigen binding fragment thereof that binds to target and has reduced immunogenicity.

In respect of the methods above, in one embodiment, said variable region is a heavy chain variable region or light chain variable region.

In one embodiment, the one or more amino acid residue is located in a CDR1, 2 or 3 region or within 20, e.g. 10 amino acid residues of a framework region adjacent to a CDR1, 2 or 3 region.

In one embodiment, the one or more amino acid residue is located in a CDR1, 2 or 3 region or within 1, 2, 3, 4 or 5 amino acid residues of a framework region adjacent to a CDR1, 2 or 3 region.

In one embodiment, the one or more amino acid residue is located in a CDR1, 2 or 3 region.

In one embodiment, the one or more amino acid residue is replaced with N.

In one embodiment, the one or more amino acid residue is replaced with T.

In one embodiment, the one or more amino acid residue is replaced with S.

In one embodiment, crystal structures of the antibody in complex with its target may be obtained so as to identify candidate amino acids to mutate and subsequently modify, for example with the addition of N-glycans. Many antibodies have been characterised in the art and thus crystal structures can readily be obtained. Alternatively, crystal structures can be determined. Further, as described elsewhere herein, methods are available in the art to determine which residues of an antibody or antigen binding fragment thereof are involved in the binding of ADAs. These methods include the use of enzyme-linked immunosorbent assay (ELISA), electrochemiluminescence immunoassay (ECLIA) methods, surface plasmon resonance (SPR) and bio-layer interferometry (BLI) and High-pressure liquid chromatography (HPLC)-based methods.

Methods for mutating amino acid sequences are well known in the art. In order to produce antibodies, monoclonal antibodies or antigen binding fragments thereof, which are co- or post-translationally modified as desired, for example via the addition of an N-linked oligosaccharide, it is envisaged that the mammalian cells are capable of inferring the correct type and pattern of oligosaccharides onto the amino acid sequence of the antibodies, monoclonal antibodies or antigen binding fragments thereof. Therefore, in some embodiments, the cells of the present invention are appropriately engineered to have a modified N-glycosylation pathway. Many such genetic modifications may be made such that the cells may express certain enzymes required to generate the required modifications. Genetic engineering of cells, including knock-outs and knock-ins of genes encoding enzymes that are involved in certain modification or processing pathways, such as the N-glycosylation pathway, may be carried out to enable these cells to produce a certain modification, such as the desired N-glycan profile. For instance, nucleic acids encoding the antibodies described herein may be expressed in mammalian (e.g., CHO) cells which have been engineered to achieve the desired modifications on the amino acid sequence of the antibody.

Any suitable methods which will be known in the art may be utilised in order to modify an amino acid sequence via mutation, co-/post-translational modifications and co-/post-translational processing (including enzymatic synthesis of glycans). For example, antibody variants into which the N-X-S and/or N-X-T sequence motifs have been introduced may be generated using site directed mutagenesis. Genes encoding the variant antibodies may be cloned into expression vectors. In one embodiment, standard cloning techniques may be used. Expression vectors may be expressed in cell lines in order to produce the antibodies, which may subsequently be collected, for example via harvesting the supernatant of the cell culture. Further, as an example of modifying antibody sequences to comprise N-linked oligosaccharides, antibodies (mutated or unmodified) may be expressed in cells capable of carrying out these modifications, for example cells which express certain oligosaccharyltransferases or glycosyltransferases.

A suitable method is further described in detail in example 1.

The invention also relates to an antibody or antigen binding fragment obtained or obtainable by a method described above.

### Exemplary therapeutic applications of the antibody or antigen binding fragment thereof

In another aspect, there is provided a pharmaceutical composition comprising an antibody or antigen binding fragment thereof of the invention and optionally a pharmaceutically acceptable carrier. The terms composition and formulation are used interchangeably herein. An antibody or antigen binding fragment thereof or the pharmaceutical composition of the invention can be administered by any convenient route, including but not limited to oral, topical, parenteral, sublingual, rectal, vaginal, ocular, intranasal, pulmonary, intradermal, intravitreal, intramuscular, intraperitoneal, intravenous, subcutaneous, intracerebral, transdermal, transmucosal, by inhalation, or topical, particularly to the ears, nose, eyes, or skin or by inhalation.

Parenteral administration includes, for example, intravenous, intramuscular, intraarterial, intraperitoneal, intranasal, rectal, intravesical, intradermal, topical or subcutaneous administration. Preferably, the compositions are administered parenterally.

The pharmaceutically acceptable carrier or vehicle can be particulate, so that the compositions are, for example, in tablet or powder form. The term "carrier" refers to a diluent, adjuvant or excipient, with which an antibody or antigen binding fragment thereof of the present invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to an animal, the antibody or antigen binding fragment thereof of the present invention or compositions and pharmaceutically acceptable carriers are sterile. Water is a preferred carrier when the binding molecule or an antibody or antigen binding fragment thereof of the present invention is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The pharmaceutical composition of the invention can be in the form of a liquid, e.g., a solution, emulsion or suspension. The liquid can be useful for delivery by injection, infusion (e.g., IV infusion) or subcutaneously.

When intended for oral administration, the composition is preferably in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as carboxymethylcellulose, ethyl cellulose, microcrystalline cellulose, or gelatin; excipients such as starch, lactose or dextrins, disintegrating agents such as alginic acid, sodium alginate, corn starch and the like; lubricants such as magnesium stearate; glidants such as colloidal silicon dioxide; sweetening agents such as sucrose or saccharin; a flavoring agent such as peppermint, methyl salicylate or orange flavoring; and a coloring agent. When the composition is in the form of a capsule (e. g. a gelatin capsule), it can contain, in addition to materials of the above type, a liquid carrier such as polyethylene glycol, cyclodextrin or a fatty oil.

The composition can be in the form of a liquid, e. g. an elixir, syrup, solution, emulsion or suspension. The liquid can be useful for oral administration or for delivery by injection. When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included.

Compositions can take the form of one or more dosage units.

In specific embodiments, it can be desirable to administer the composition locally to the area in need of treatment, or by intravenous injection or infusion.

The amount of the therapeutic that is effective/active in the treatment of a particular disorder or condition will depend on the nature of the disorder or condition and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances. Factors like age, body weight, sex, diet, time of administration, rate of excretion, condition of the host, drug combinations, reaction sensitivities and severity of the disease shall be taken into account.

Typically, the amount is at least about 0.01% of an antibody or antigen binding fragment thereof of the present invention by weight of the composition. When intended for oral administration, this amount can be varied to range from about 0.1% to about 80% by weight of the composition. Oral compositions can comprise from about 4% to about 50% of the antibody or antigen binding fragment thereof of the present invention by weight of the composition.

Compositions of the present invention can be prepared so that a parenteral dosage unit contains from about 0.01 % to about 2% by weight of the antibody or antigen binding fragment thereof of the present invention. The invention also relates to a device, such as a pre-filled syringe which comprises an antibody or antigen binding fragment thereof of the invention.

For administration by injection, the composition can comprise from about typically about 0.1 mg/kg to about 250 mg/kg of the subject's body weight, preferably, between about 0.1 mg/kg and about 20 mg/kg of the subject's body weight, and more preferably about 1 mg/kg to about 10 mg/kg of the subject's body weight. In one embodiment, the composition is administered at a dose of about 1 to 30 mg/kg, e.g., about 5 to 25 mg/kg, about 10 to 20 mg/kg, about 1 to 5 mg/kg, or about 3 mg/kg. The dosing schedule can vary from e.g., once a week to once every 2, 3, or 4 weeks.

As used herein, "treat", "treating" or "treatment" means inhibiting or relieving a disease or disorder. For example, treatment can include a postponement of development of the symptoms associated with a disease or disorder, and/or a reduction in the severity of such symptoms that will, or are expected, to develop with said disease. The terms include ameliorating existing symptoms, preventing additional symptoms, and ameliorating or preventing the underlying causes of such symptoms. Thus, the terms denote that a beneficial result is being conferred on at least some of the mammals, e.g., human patients, being treated. Many medical treatments are effective for some, but not all, patients that undergo the treatment.

The term "subject" or "patient" refers to an animal which is the object of treatment, observation, or experiment. By way of example only, a subject includes, but is not limited to, a mammal, including, but not limited to, a human or a non-human mammal, such as a non-human primate, murine, bovine, equine, canine, ovine, or feline.

As used herein, the term "effective amount" means an amount of the antibody or antigen binding fragment thereof as described herein, that when administered alone or in combination with an additional therapeutic agent to a cell, tissue, or subject, is effective to achieve the desired therapeutic or prophylactic effect under the conditions of administration.

### Methods of treatment using the antibody or antigen binding fragment thereof of the invention

A skilled person will understand that the antibody or antigen binding fragment thereof of the invention can be used to treat a multitude of disease, depending on the therapeutic target of the antibody. Therapeutic antibodies are well known and for example discussed in Sharma et al Molecules. 2023 Sep 5;28(18):6438.

In another aspect, the invention relates to an antibody or antigen binding fragment thereof or a pharmaceutical composition according to the invention for use in the treatment of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases.

In another aspect, the invention relates to a method of treating cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases comprising administering antibody or antigen binding fragment thereof a pharmaceutical composition according to the invention.

The invention also relates to the use of an antibody or antigen binding fragment thereof according to the invention in the manufacture of a medicament for the treatment of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological conditions or transplantation associated diseases

An inflammatory disease may be rheumatoid arthritis, psoriatic arthritis, ankylosing spondylitis, dermatitis, Crohn's disease, plaque psoriasis or ulcerative colitis.

A hematology disease may be hemophilia A or sickle cell disease.

A pulmonary disease may be asthma, lung cancer, and respiratory infections, COPD, respiratory syncytial virus (RSV) or IPF.

An infectious disease may be HIV, tuberculosis or malaria.

A neurological disorder may be multiple sclerosis (MS), Alzheimers or Parkinsons.

A metabolic disorder may be Pompe disease.

An ophthalmic disease may be age-related macular degeneration.

A bone and joint disease may be osteoporosis.

A dermatological condition may be skin diseases such as psoriasis or atopic dermatitis.

In one embodiment, the disease is selected from psoriasis, systemic lupus erythematosis, rheumatoid arthritis, osteoarthritis, juvenile chronic arthritis, spondyloarthropathies, systemic sclerosis, idiopathic inflammatory myopathies, Sjogren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain Barre syndrome, chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, autoimmune haematological disorders (including e.g. hemolytic anaemia, aplastic anaemia, pure red cell anaemia and idiopathic thrombocytopenia), autoimmune inflammatory bowel disease (including e.g. ulcerative colitis, Crohn's disease and Irritable Bowel Syndrome), transplantation associated diseases including graft rejection and graft-versus-host-disease.

In one embodiment, the disease is cancer and the invention thus relates to methods for the prevention and/or treatment of cancer, comprising administering to a subject a cell or cell population comprising an antibody or antigen binding fragment thereof as described herein, said method comprising administering, to a subject in need thereof, a pharmaceutically active amount of an antibody or antigen binding fragment thereof or pharmaceutical composition of the invention. The invention also relates to an antibody or antigen binding fragment thereof or pharmaceutical composition according to the invention for use in therapy. The invention also relates to an antibody or antigen binding fragment thereof or pharmaceutical composition according to the invention as described herein for use in the treatment of cancer. The invention also relates to the use of an antibody or antigen binding fragment thereof or pharmaceutical composition according to the invention in the manufacture of a medicament for the treatment of cancer.

The term "cancer" refers to a disease characterized by the uncontrolled growth of aberrant cells. Cancer includes all types of cancerous growths or oncogenic processes, metastatic tissues or malignantly transformed cells, tissues or organs irrespective of the histopathologic type or stage of invasiveness. Cancer cells can spread locally or through the bloodstream and lymphatic system to other parts of the body. The cancer may be a primary or a secondary cancer. Thus, a molecule as described herein may be for use in a method of treating cancer in an individual, wherein the cancer is a primary tumour and/or a tumour metastasis.

In one embodiment, the cancer is selected from a haematological cancer or malignancy or a solid tumor. Hematologic cancers are cancers of the blood or bone marrow. Solid tumors are abnormal masses of tissue that usually do not contain cysts or liquid areas.

The cancer to be treated using an antibody or antigen binding fragment thereof or pharmaceutical composition of the invention may be a solid cancer. Examples of various cancers are described further herein and include, but are not limited to, mesothelioma, breast cancer, ovarian cancer, cervical cancer, pancreatic cancer, colorectal cancer, renal cancer, liver cancer, lung cancer, and prostate cancer.

In one embodiment, the cancer is metastatic.

In particular, cancers that may be treated by methods, uses, an antibody or antigen binding fragment thereof and compositions described herein include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In reference to cancer, an effective amount may comprise an amount sufficient to cause a tumour to shrink and/or to decrease the growth rate of the tumour (such as to suppress tumour growth) or to prevent or delay other unwanted cell proliferation. In some embodiments, an effective amount is an amount sufficient to delay development or prolong survival or induce stabilisation of the cancer or tumour. In some embodiments, a therapeutically effective amount is an amount sufficient to prevent or delay recurrence. For example, a "therapeutically effective dosage" may induce tumour shrinkage by at least about 5% relative to baseline measurement, such as at least about 10%, or about 20%, or about 60% or more. The baseline measurement may be derived from untreated subjects.

In reference to an autoimmune disease, an effective amount may comprise an amount sufficient to partially or fully prevent disease activity, or to prevent or delay new disease activity or flare ups.

In some embodiments, an effective amount is an amount sufficient to delay the development of symptoms or prolong symptom-free periods or induce stabilisation of the disease.

In some embodiments, a therapeutically effective amount is an amount sufficient to prevent or delay recurrence of the disease, flare ups of the disease or incidences of new disease activity.

An invention method may not take effect immediately. For example, treatment may be followed by an increase in disease activity, but over time eventual stabilization or reduction in disease activity in a given subject may subsequently occur.

Additional adverse symptoms and complications associated with neoplasia and autoimmune disease that can be inhibited, reduced, decreased, delayed, or prevented include, for example, nausea, swollen glands, lack of appetite, lethargy, pain, swelling, and discomfort. Thus, a partial or complete decrease or reduction in the severity, duration or frequency of an adverse symptom or complication associated with or caused by a cellular hyperproliferative disorder, an improvement in the subject's quality of life and/or well-being, such as increased energy, appetite, psychological well-being, are all particular non-limiting examples of therapeutic benefit.

A therapeutic benefit or improvement therefore can also include a subjective improvement in the quality of life of a treated subject. In an additional embodiment, the invention prolongs or extends lifespan (survival) of the subject. In a further embodiment, a method improves the quality of life of the subject.

### Exemplary combinations with other agents

An antibody or antigen binding fragment thereof or pharmaceutical composition of the invention may be administered as the sole active ingredient or in combination with one or more other therapeutic agent, e.g. an anti-cancer therapy. A therapeutic agent is a compound or molecule which is useful in the treatment of a disease. Examples of therapeutic agents include antibodies, antibody fragments, drugs, toxins, nucleases, hormones, immunomodulators, pro-apoptotic agents, anti-angiogenic agents, boron compounds, photoactive agents or dyes and radioisotopes. An antibody molecule includes a full antibody or fragment thereof (e.g., a Fab, F(ab')2, Fv, a single chain Fv fragment (scFv) or a single domain antibody, for example a V_{H} domain, antibody mimetic protein.

The anti-cancer therapy may include a therapeutic agent or radiation therapy and includes gene therapy, viral therapy, RNA therapy bone marrow transplantation, nanotherapy, targeted anti-cancer therapies or oncolytic drugs. Examples of other therapeutic agents include checkpoint inhibitors, antineoplastic agents, immunogenic agents, attenuated cancerous cells, tumor antigens, antigen presenting cells such as dendritic cells pulsed with tumor-derived antigen or nucleic acids, immune stimulating cytokines (e.g., IL-2, IFNa2, GM-CSF), targeted small molecules and biological molecules (such as components of signal transduction pathways, e.g. modulators of tyrosine kinases and inhibitors of receptor tyrosine kinases, and agents that bind to tumor- specific antigens, including EGFR antagonists), an anti-inflammatory agent, a cytotoxic agent, a radiotoxic agent, or an immunosuppressive agent and cells transfected with a gene encoding an immune stimulating cytokine (e.g., GM-CSF) or chemotherapy. In another specific embodiment, the chemotherapeutic agent or radiation therapy is administered prior or subsequent to administration of the composition of the present invention, preferably at least an hour, five hours, 12 hours, a day, a week, a month, more preferably several months (e. g. up to three months), prior or subsequent to administration of composition of the present invention.

With reference to checkpoint inhibitors, checkpoint molecules include PD-1, PDL-1, PDL-2, LAG-3, CTLA-4, TIGIT and TIM-3. Checkpoint inhibitors include PD-1 inhibitors (Nivolumab, Pembrolizumab, and Cemiplimab), PDL-1 inhibitors (Atezolimumab, Durvalumab and Avelumab), and CTLA-4 inhibitor (Ipilimumab).

In one embodiment, administration is in combination with surgery. An antibody or antigen binding fragment thereof or pharmaceutical composition of the invention may be administered at the same time or at a different time as the other therapy, e.g., simultaneously, separately or sequentially.

### Exemplary kits

In another aspect, the invention provides a kit comprising antibody or antigen binding fragment thereof of the invention. The kit may also comprise instructions for use. The kits may include a labeled antibody or antigen binding fragment thereof of the invention as described above and one or more compounds for detecting the label. Also provided is an antibody or antigen binding fragment thereof of the invention packaged in lyophilized form or packaged in an aqueous medium. The kits may include a reagent, (e.g. for reconstituting) and / or instructions for use and/or a device for administration.

Unless otherwise defined herein, scientific and technical terms used in connection with the present disclosure shall have the meanings that are commonly understood by those of ordinary skill in the art. While the foregoing disclosure provides a general description of the subject matter encompassed within the scope of the present disclosure, including methods, as well as the best mode thereof, of making and using this disclosure, the following examples are provided to further enable those skilled in the art to practice this disclosure. However, those skilled in the art will appreciate that the specifics of these examples should not be read as limiting on the invention, the scope of which should be apprehended from the claims and equivalents thereof appended to this disclosure. Various further aspects and embodiments of the present disclosure will be apparent to those skilled in the art in view of the present disclosure.

All documents mentioned in this specification are incorporated herein by reference in their entirety, including references to gene accession numbers, scientific publications and references to patent publications.
"and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein. Unless context dictates otherwise, the descriptions and definitions of the features set out above are not limited to any particular aspect or embodiment of the invention and apply equally to all aspects and embodiments which are described.

The invention is also described in the following embodiments.
1. An antibody or antigen binding fragment thereof with reduced ADA binding comprising a modified amino acid sequence in the variable region wherein said amino acid sequence is modified to comprise one or more N-X-S and/or N-X-T sequence motifs wherein X is any amino acid other than proline.
2. The antibody or antigen binding fragment thereof according to embodiment 1 wherein a CDR1, 2 or 3 region or a region within 20, e.g. 10 amino acid residues of a framework region adjacent to a CDR1, 2 or 3 region is modified.
3. The antibody or antigen binding fragment thereof according to embodiment 1 or 2 wherein one or more amino acid residue located in a variable region is substituted with N, T or S.
4. The antibody or antigen binding fragment thereof according to embodiment 1 or 2 wherein a N-X-SΓΓ sequence motif is inserted into an amino acid residue sequence.
5. The antibody or antigen binding fragment thereof according to embodiment 4 wherein a CDR1, 2 or 3 region or a region or within 1, 2, 3, 4 or 5 amino acid residues of a framework region adjacent to a CDR1, 2 or 3 region is modified.
6. The antibody or antigen binding fragment thereof according to a preceding embodiment wherein a CDR1, 2 or 3 region is modified.
7. The antibody or antigen binding fragment thereof according to a preceding embodiment wherein said one or more motif is located in an epitope for binding ADAs or in proximity thereto.
8. The antibody or antigen binding fragment thereof according to embodiment 3 wherein the one or more amino acid residue is substituted with N.
9. The antibody or antigen binding fragment thereof according to embodiment 3 wherein the one or more amino acid residue is substituted with T.
10. The antibody or antigen binding fragment thereof according to embodiment 3 wherein the one or more amino acid residue is replaced with S.
11. The antibody or antigen binding fragment thereof according to a preceding embodiment wherein said variable region is a heavy chain variable region or light chain variable region.
12. The antibody or antigen binding fragment thereof according to any of embodiments 2 to 11 wherein the epitope is a linear epitope or a confirmational epitope.
13. The antibody or antigen binding fragment thereof according to a preceding embodiment wherein said fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.
14. The antibody or antigen binding fragment thereof according to a preceding embodiment wherein said antibody or fragment is conjugated to another moiety.
15. The antibody or antigen binding fragment thereof according to embodiment 14 wherein the other moiety is a therapeutic moiety, half-life extending moiety, toxin or label.
16. The antibody or antigen binding fragment thereof according to a preceding embodiment wherein said antibody or antigen binding fragment thereof is N-glycosylated at the N-X-S/T sequence motif.
17. A nucleic acid encoding an antibody or antigen binding fragment thereof according to a preceding embodiment.
18. A vector comprising a nucleic acid according to embodiment 17.
19. A host cell comprising a nucleic acid according to embodiment 17 or a vector according to embodiment 18.
20. A pharmaceutical composition comprising an antibody or antigen binding fragment thereof according to any of embodiments 1 to 16 and a pharmaceutically acceptable excipient.
21. An antibody or antigen binding fragment thereof according to any of embodiments 1 to 16 or a pharmaceutical composition according to embodiment 20 for use in the treatment of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases.
22. A method of treating of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases comprising administering antibody or antigen binding fragment thereof according to any of embodiments 1 to 16 or a pharmaceutical composition according to embodiment 20 to a subject.
23. The antibody or antigen binding fragment thereof for use according to embodiment 21 or the method according to embodiment 22 wherein said antibody or antigen binding fragment thereof is administered together with another therapy.
24. A method for producing an antibody or antigen binding fragment thereof with reduced ADA binding comprising modifying the amino acid sequence of the variable region to comprise one or more N-X-S and/or N-X-T sequence motifs wherein X is any amino acid other than proline.
25. The method of embodiment 24 wherein said one or more motif is located in an epitope for antigen binding or in proximity thereto.
26. The method of embodiment 24 or 25 wherein one or more amino acid residue located in a variable region is substituted with N, T or S to introduce a N-X-S/T sequence motif.
27. The method of any of embodiments 24 to 26 wherein a N-X-S/T sequence motif is inserted into an amino acid residue sequence located in an epitope for antigen binding or in proximity thereto.
28. The method of any of embodiments 24 to 27 comprising the steps of
   a) substituting one or more amino acid residue in a variable region with a N, S or T to introduce a N-X-S/T sequence motif and wherein said one or more amino acid residue is located in an epitope for antigen binding and
   b) expressing a nucleotide sequence or nucleic acid encoding the modified antibody or antigen binding fragment thereof in a suitable host cell or host organism.
29. A method for reducing the ADA response of an antibody or antigen binding fragment thereof with reduced immunogenicity comprising modifying the amino acid sequence of the variable region to comprise one or more N-X-S and/or N-X-T sequence motifs wherein X is any amino acid other than proline.
30. The method of embodiment 29 wherein said one or more motif is located in an epitope for antigen binding or in proximity thereto.
31. The method of embodiment 29 or 30 wherein one or more amino acid residue located in a variable region is substituted with N, T or S to introduce a N-X-S/T sequence motif.
32. The method of any of embodiments 29 to 31 wherein a N-X-S/T sequence motif is inserted into an amino acid residue sequence located in an epitope for antigen binding or in proximity thereto.
33. The method of any of embodiments 29 to 32 comprising the steps of
   a) substituting one or more amino acid residue in a variable region with a N, S or T to introduce a N-X-S/T sequence motif and wherein said one or more amino acid residue is located in an epitope for antigen binding and
   b) expressing a nucleotide sequence or nucleic acid encoding the modified antibody or antigen binding fragment thereof in a suitable host cell or host organism.
34. The method of any of embodiments 24 to 33 wherein said variable region is a heavy chain variable region or light chain variable region.
35. The method of any of embodiments 24 to 34 wherein the one or more amino acid residue is located in a CDR1, 2 or 3 region or within 20, e.g.10 amino acid residues of a framework region adjacent to a CDR1, 2 or 3 region.
36. The method of any of embodiment 35 wherein the one or more amino acid residue is located in a CDR1, 2 or 3 region or within 1, 2, 3, 4 or 5 amino acid residues of a framework region adjacent to a CDR1, 2 or 3 region.
37. The method of any of embodiments 35 wherein the one or more amino acid residue is located in a CDR1, 2 or 3 region.
38. The method of embodiment 33 wherein the one or more amino acid residue is substituted with N.
39. The method of embodiment 33 wherein the one or more amino acid residue is substituted with T.
40. The method of embodiment 33 wherein the one or more amino acid residue is substituted with S.
41. The method of any of embodiments 24 to 40 comprising the step of assessing binding of the antibody or antigen binding fragment thereof to its target antigen and/or assessing binding ADAs to the antibody or antigen binding fragment thereof.
42. The method of any of embodiments 24 to 41 comprising the step of determining which one or more residue in the variable region is located in an epitope for antigen binding.
43. The method of any of embodiments 24 to 42 comprising the step of determining which one or more residue in the variable region is located in an epitope for ADA binding.
44. The method of any of embodiments 24 to 43 wherein the epitope is a linear epitope or a confirmational epitope.
45. The method of any of embodiments 24 to 44 wherein said fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.
46. A kit comprising antibody or antigen binding fragment thereof according to any of embodiments 1 to 16 or 20 and optionally instructions for use.
47. A binding molecule comprising antibody or antigen binding fragment thereof according to any of embodiments 1 to 16.
48. A binding molecule according to embodiment 47 wherein said binding molecule comprises a second antibody or antigen binding fragment thereof which binds to the same or a different target.

The invention is now further described in the following non-limiting examples.

### EXAMPLES

### Example 1- Method for identifying novel N-glycan sites to block ADA binding

Described here is a method for identifying optimal sites to introduce novel N-glycosylation sites in the Fab region of monoclonal antibodies (mAbs) to block anti-drug antibody (ADA) binding. The method involves first identifying key amino acid residues that are critical for the interaction between the antibody and its therapeutic target. These residues are typically found within or adjacent to the complementarity-determining region (CDR), the region of the antibody that engages the antigen. Once the critical residues involved in target binding are mapped, the next step is to analyze the surrounding amino acid sequences, focusing on a window of 20 amino acids on either side of these key residues. This 40-amino-acid window is selected due to its proximity to regions targeted by ADAs and its ability to accommodate N-glycans that effectively block ADA binding.

In this method, each amino acid within this defined window is systematically mutated one at a time to create potential N-glycosylation sites. Specifically, mutations are introduced to form the consensus N-glycosylation sequon (Asn-X-Ser/Thr), where "X" refers to any amino acid except proline. This sequon is essential for enzymatic attachment of N-glycans during protein expression. By generating a series of mutations along the Fab region, specifically within the CDR, we create novel N-glycosylation sites at various positions relative to the key residues involved in target binding. These mutations are designed to place the N-glycan in such a way that it can sterically block or shield epitopes that are recognized by ADAs, thus reducing the likelihood of immune recognition and neutralization of the therapeutic antibody.

Once the novel N-glycosylation sites are engineered, each variant is assessed for its ability to bind the therapeutic target. This is done through structural modeling, binding assays, and biophysical characterization to ensure that the introduced N-glycan does not interfere with antigen binding. In parallel, ADA binding assays are conducted to determine whether the new glycan sites effectively reduce or eliminate ADA interactions. This iterative process allows for the identification of novel N-glycan positions that optimally block ADA binding without compromising the therapeutic activity of the mAb.

This method is advantageous as it focuses on regions of the mAb that are most likely to impact immunogenicity without disrupting the structural integrity or binding affinity of the mAb. By systematically exploring a range of potential N-glycosylation sites near the target-binding interface, the method provides a rational and efficient approach for glycoengineering mAbs to reduce immunogenicity. Moreover, this approach allows for the fine-tuning of N-glycan placement to achieve optimal immune shielding, providing a valuable tool for developing next-generation therapeutic mAbs with enhanced safety and efficacy profiles.

In the following examples, the invention is illustrated using adalimumab, an antibody that binds TNF-alpha. To identify the novel N-glycan sites on adalimumab, the present inventors used the crystal structure (PDB ID: 3WD5) of the Fab domain of adalimumab in complex with soluble TNF. The considerations for selecting novel N-glycan sites were as follows:
1. Asn or Ser/Thr residue that were already in place;
2. Asn residues to be glycosylated that were solvent exposed, calculated *in silico;*
3. Novel N-glycan sites located in a linker region or disordered region so as to not disrupt proper folding; and
4. Novel N-glycan sites facing away from active or protein-protein interaction domains.

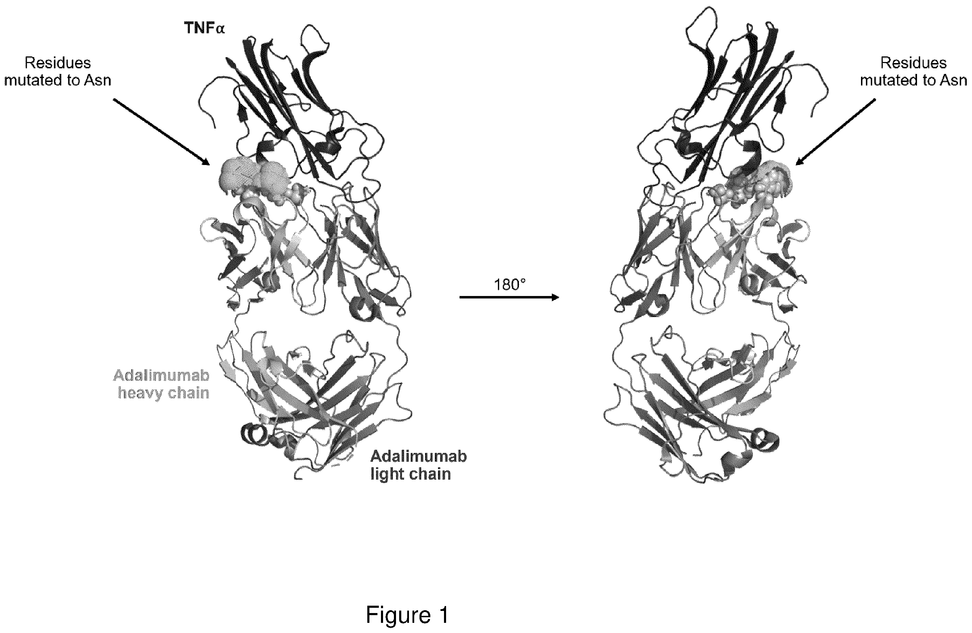

Figure 1 shows a glycovariant design of adalimumab showing a number of amino acid sites that may be mutated to an asparagine residue in order to create an N-X-S/T sequence motif. The modified VH regions are shown in sequences are SEQ ID No. 3, 4, 5 and 6. The modified residues are W53, Y101, L102 and A105. The numbering is with reference to the wild type VH sequence of adalimumab as shown in SEQ ID NO. 1.

### Example 2 - Expression of adalimumab glycovariants

Once the novel N-glycan sites on adalimumab were identified, the genes encoding for adalimumab were cloned into mammalian expression vectors - pFUSE2ss-CLIg-hK and pFUSEss-CHIg-hG1 - using standard cloning techniques. Glycovariants of adalimumab were obtained by performing site specific mutagenesis. Using PEI-mediated transfection, the plasmids were transfected into CHO-S cells and transient expression of each adalimumab glycovariant occurred for 7 days at 37 °C. Following cell culture, cell cultures were centrifuged so that the supernatant of each transfection could be harvested. Protein expression (proteins in the supernatant) was monitored by SDS-PAGE.

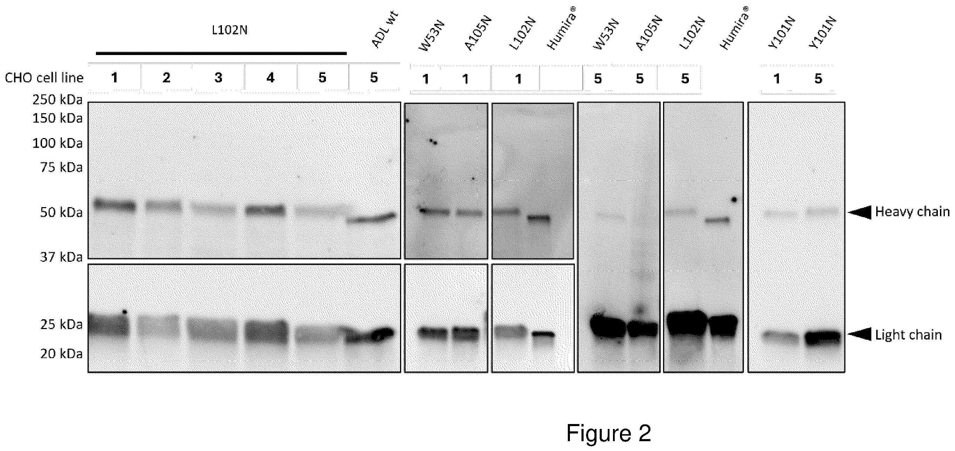

An increased molecular weight was observed for the glycovariants indicating increase in the number of N-glycans. Figure 2 shows the expression of W53N, Y101N, L102N, and A105N (Figure 2) in genetically engineered CHO cells.

### Example 3 - PNGase F assay

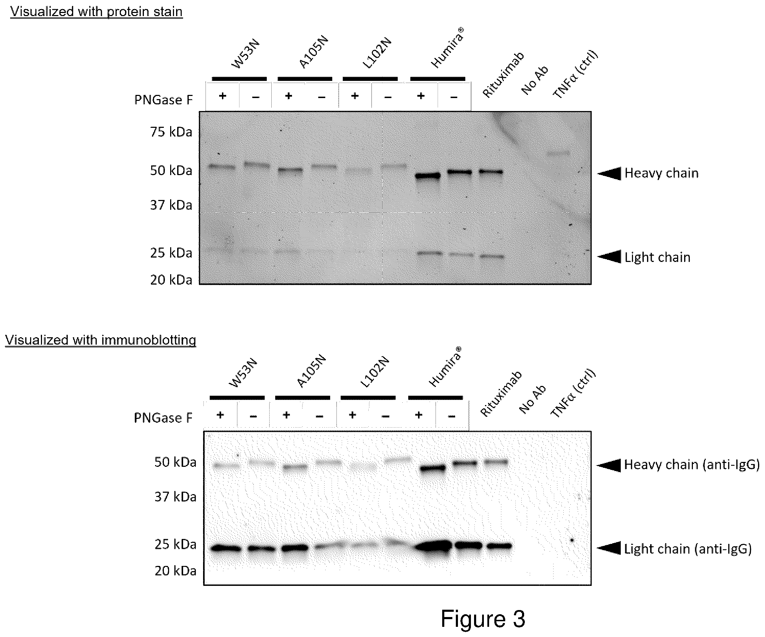

Using the supernatant harvested after expression, 26 µL of each supernatant was subjected to PNGase F digestion, which cleaves N-glycans from the surface of the protein. Briefly, after denaturation proteins were incubated at 37 °C overnight with 0.5 µL of PNGase F. The samples were subsequently boiled at 100 °C in Laemmli buffer and analysed using SDS-PAGE. Changes in molecular weight as seen using SDS-PAGE indicate that the additional molecular weight of the glycovariants is a result of an increase in number of N-glycans. A Western blot was performed using anti-human IgG to observe adalimumab (Figure 3). Figure 3 shows that the increase in molecular weight of the proteins is due to the addition of N-glycans.

### Example 4 - TNF-alpha binding assay (pulldown)

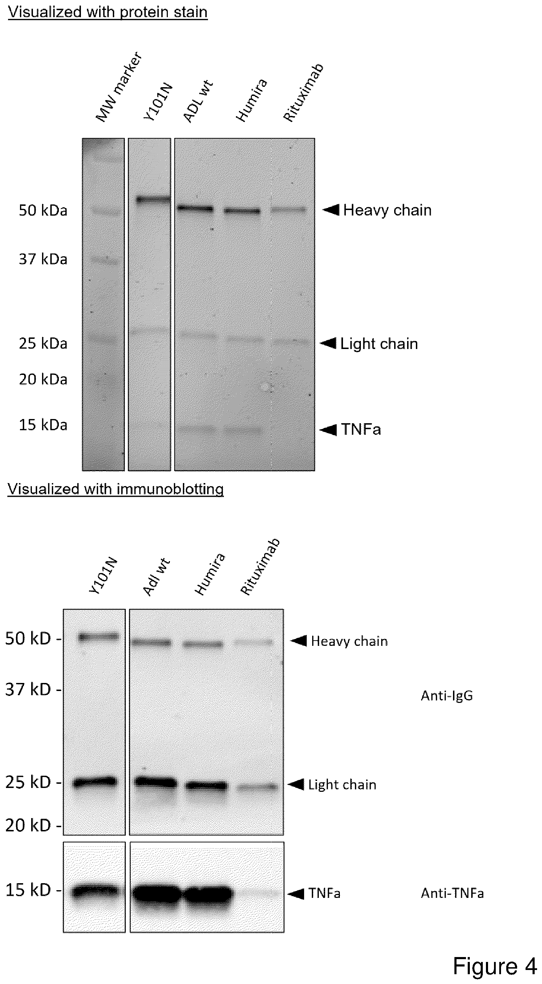

Using the supernatant harvested after expression, 1 µg (50 to 100 µL of SN) of adalimumab and the adalimumab variants were immobilized on Protein A agarose beads for 30 minutes. The beads were subsequently washed 3X 5 minutes in PBS 0.05% Tween shaking at room temperature. 1 µg of purified TNF was incubated with the immobilized adalimumab for 30 minutes. The beads were subsequently washed 3X 5 minutes in PBS 0.05% Tween shaking at room temperature. The samples were boiled at 100 °C in Laemmli buffer and analysed using SDS-PAGE. A Western blot was performed using anti-TNF to observe the TNF binding to adalimumab. Figure 4 shows representative results of the TNFα binding assay and shows the adalimumab variant Y101N interacting with TNFα.

### Example 5 - monoclonal ADA binding (pulldown)

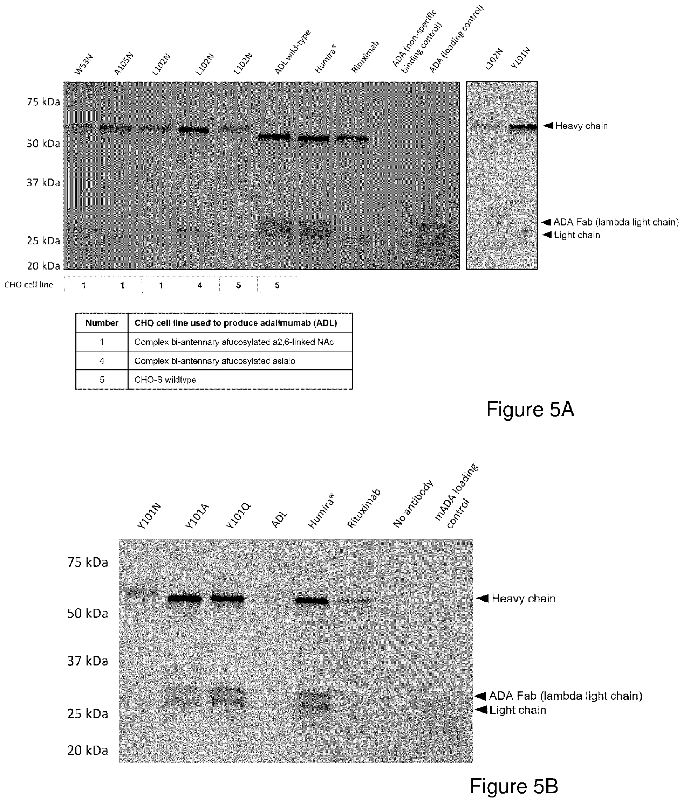

Using the supernatant harvested after expression, 1 to 2 µg (50 µL to 150 µL of supernatant) of adalimumab and the adalimumab variants were immobilized on Protein A agarose beads for 20 minutes. The beads were subsequently washed 3X 5 minutes in PBS 0.05% Tween shaking at room temperature. 3 µg of monoclonal ADA (Fab fragment with a Flag tag) was incubated with the immobilized adalimumab for 30 minutes. The beads were subsequently washed 3X 5 minutes in PBS 0.05% Tween shaking at room temperature. The samples were boiled at 100 °C in Laemmli buffer and analysed using SDS-PAGE. A Western blot was performed using anti-Flag to observe ADA binding to adalimumab. Figure 5A shows the results of the ADA binding assay, showing complete disruption of ADA binding to the W53N, Y101N, L102N, and A105N variants of adalimumab.

### Example 6 - polyclonal ADA binding (pulldown)

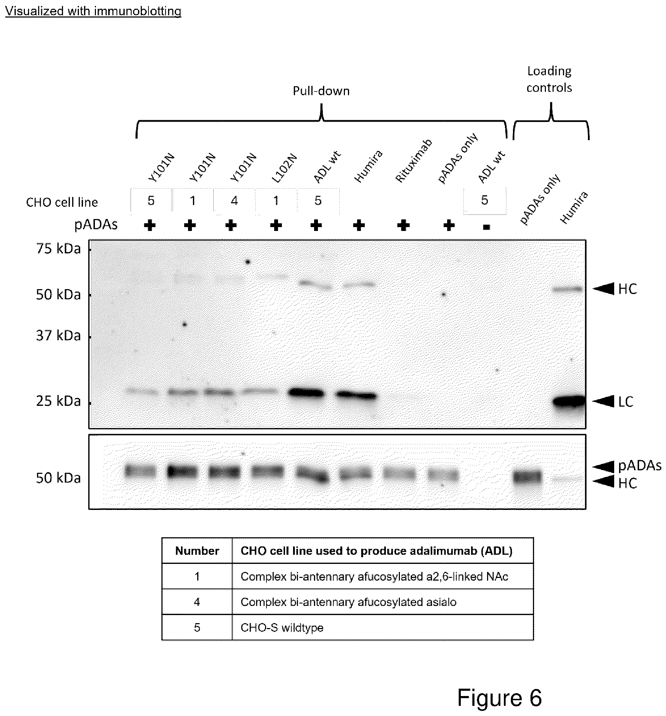

Rabbit polyclonal ADAs (1.5 to 2 ug) were immobilized on sheep anti-rabbit IgG beads for 30 minutes. The beads were subsequently washed 3× 5 minutes in PBS 2 mM EDTA, 0.1% BSAshaking at room temperature. Using the supernatant harvested after expression, 1 to 2 ug of adalimumab and the adalimumab variants were incubated with the immobilized ADAs for 30 minutes. The beads were subsequently washed 3X 5 in Laemmli buffer and analysed using SDS-PAGE. A Western blot was performed using anti-human IgG F(ab')2 to observe adalimumab binding to polyclonal ADAs. Figure 6 shows the results of the ADA binding assay, showing substantial disruption of the W53N, Y101N, L102N, and A105N variants binding to polyclonal ADAs.

### Example 7 - ADA out-competition assay (ELISA-based)

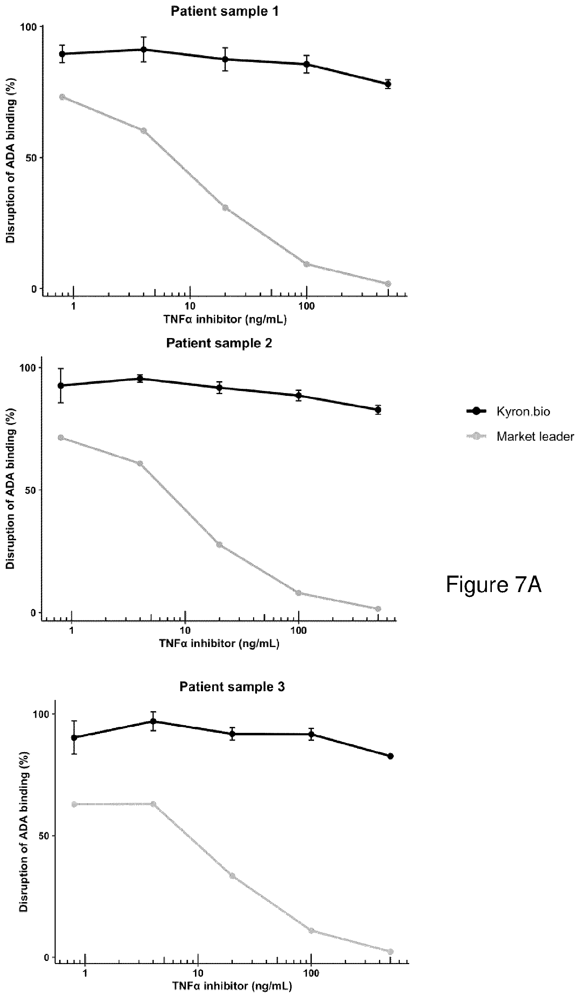

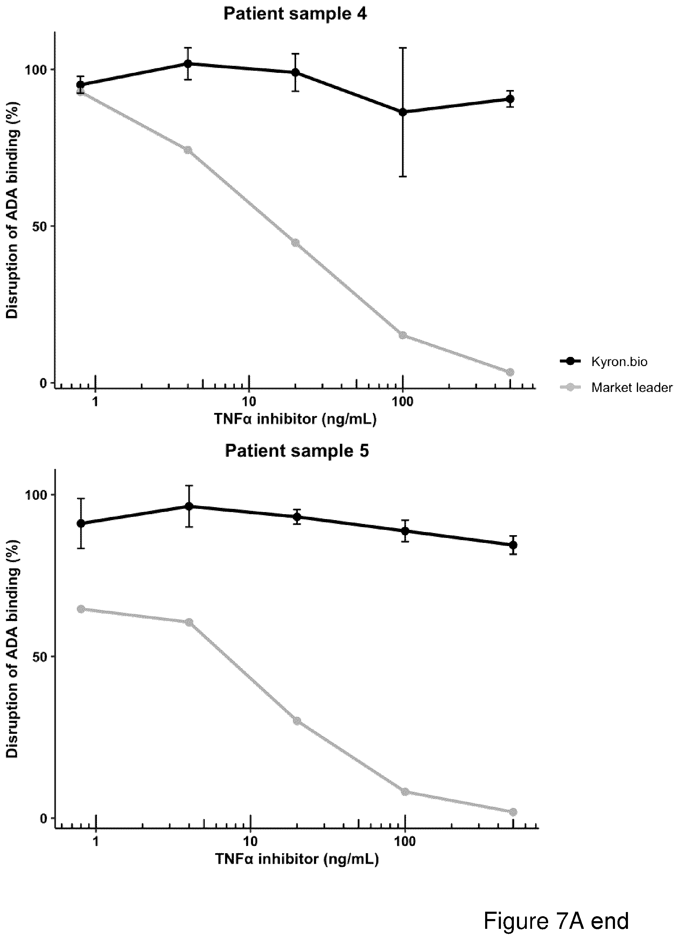

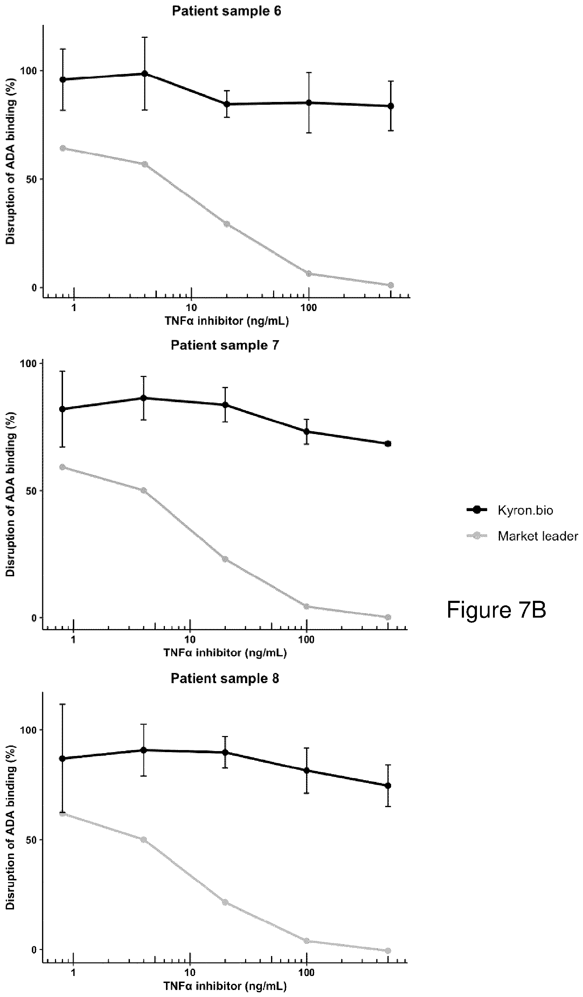

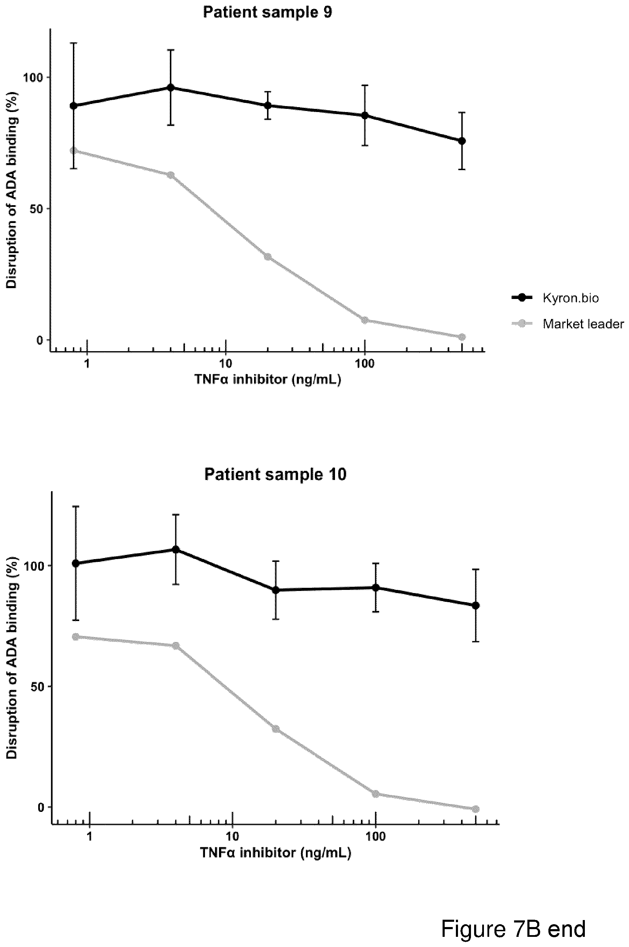

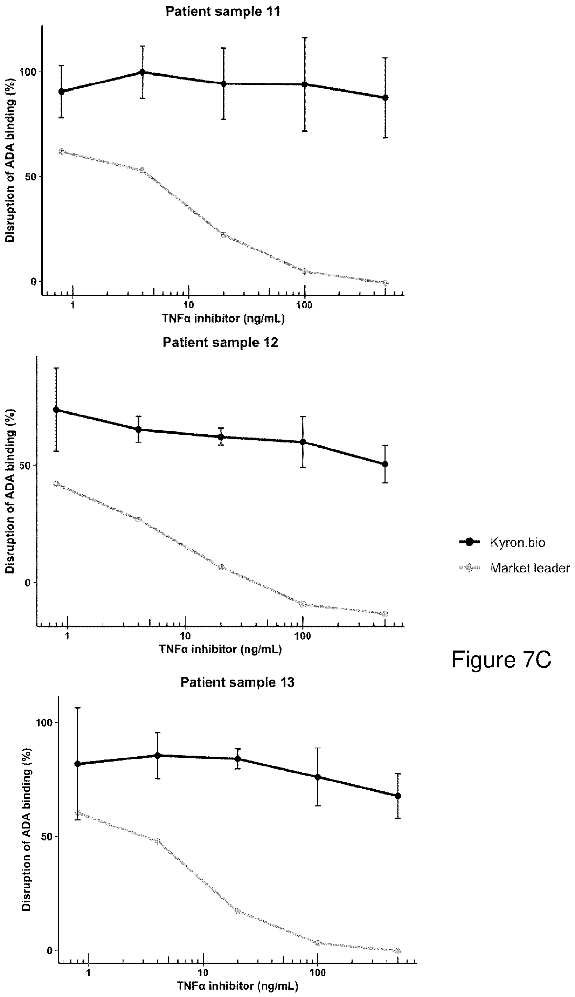

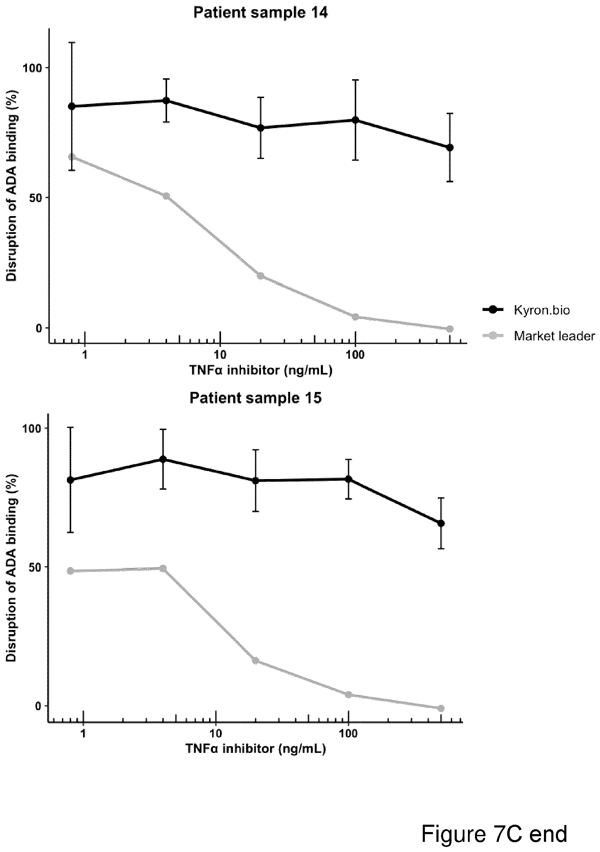

Wild-type adalimumab was immobilized in 96-well plates. Human serum from patients with ankylosing spondylitis and rheumatoid arthritis containing varying amounts of ADAs was added to each well and incubated with immobilized wild-type adalimumab. Biotinylated wild-type adalimumab was subsequently added to each well in combination with Humira^{®} or L102N anti-TNFa. Competition was assessed based on fluorescently labelled streptavidin. A decrease in fluorescence indicates binding of Humira^{®} or L102N anti-TNFa to ADAs. Figure 7 shows significant disruption of ADA binding to L102N anti-TNFa while substantial binding of ADAs to Humira^{®} remains. SEQUENCES FORMING PART OF THE DESCRIPTION
SEQ ID NO: 1 (adalimumab heavy chain) 
EVQLVESGGGLVQPGRSLRLSCAASGFTFDDYAMHWVRQAPGKGLEWVSAITWNSGHIDYADSVEGRFTI
SRDNAKNSLYLDMNSLRAEDTAVYYCAKVSYLSTASSLDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK
 Note that the constant region of the adalimumab heavy chain is shown in italics and spans amino acid residues S123 to K451 of SEQ ID NO: 1. CDRs are underlined.
SEQ ID NO: 2 (adalimumab light chain) 
DIQMTQSPSSLSASVGDRVTITCRASQGIRNYLAWYQQKPGKAPKLLIYAASTLQSGVPSRFSGSGSGTDF
TLTISSLQPEDVATYYCQRYNRAPYTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC
 Note that the constant region of the adalimumab light chain is shown in italics and spans amino acid residues R108 to C214 of SEQ ID NO: 2. CDRs are underlined.

### Variant: single mutation in the heavy chain (L102N), see residue in bold

SEQ ID NO: 3 (variant - VH) 
EVQLVESGGGLVQPGRSLRLSCAASGFTFDDYAMHWVRQAPGKGLEWVSAITWNSGHIDYADSVEGRFTISRDNAKNSL
YLDMNSLRAEDTAVYYCAKVSYNSTASSLDYWGQGTLVTVSS
SEQ ID NO: 2 (adalimumab light chain)

DIQMTQSPSSLSASVGDRVTITCRASQGIRNYLAWYQQKPGKAPKLLIYAASTLQSGVPSRFSGSGSGTDFTLTISSLQ
PEDVATYYCQRYNRAPYTFGQGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

### Variant: single mutation in the heavy chain (W53N), see residue in bold

SEQ ID NO: 4 (variant-VH) 
EVQLVESGGGLVQPGRSLRLSCAASGFTFDDYAMHWVRQAPGKGLEWVSAITNNSGHIDYADSVEGRFTISRDNAKNSL
YLDMNSLRAEDTAVYYCAKVSYLSTASSLDYWGQGTLVTVSS
SEQ ID NO: 2 (adalimumab light chain)

DIQMTQSPSSLSASVGDRVTITCRASQGIRNYLAWYQQKPGKAPKLLIYAASTLQSGVPSRFSGSGSGTDFTLTISSLQ
PEDVATYYCQRYNRAPYTFGQGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

### Variant: single mutation in the heavy chain (A105N), see residue in bold

SEQ ID NO: 5 (variant - VH) 
EVQLVESGGGLVQPGRSLRLSCAASGFTFDDYAMHWVRQAPGKGLEWVSAITWNSGHIDYADSVEGRFTISRDNAKNSL
YLDMNSLRAEDTAVYYCAKVSYLSTNSSLDYWGQGTLVTVSS
SEQ ID NO: 2 (adalimumab light chain)

DIQMTQSPSSLSASVGDRVTITCRASQGIRNYLAWYQQKPGKAPKLLIYAASTLQSGVPSRFSGSGSGTDFTLTISSLQ
PEDVATYYCQRYNRAPYTFGQGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

### Variant: single mutation in the VH (Y101N)

SEQ ID NO: 6 (variant - VH) 
EVQLVESGGGLVQPGRSLRLSCAASGFTFDDYAMHWVRQAPGKGLEWVSAITWNSGHIDYADSVEGRFTISRDNAKNSL
YLDMNSLRAEDTAVYYCAKVSNLSTASSLDYWGQGTLVTVSS
SEQ ID NO: 2 (adalimumab light chain)

DIQMTQSPSSLSASVGDRVTITCRASQGIRNYLAWYQQKPGKAPKLLIYAASTLQSGVPSRFSGSGSGTDFTLTISSLQ
PEDVATYYCQRYNRAPYTFGQGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

### REFERENCES

Anthony RM, Nimmerjahn F, Ashline DJ, Reinhold VN, Paulson JC, Ravetch JV. Recapitulation of IVIG anti-inflammatory activity with a recombinant IgG Fc. Science. 2008 Apr 18;320(5874):373-6. doi: 10.1126/science.1154315. PMID: 18420934; PMCID: PMC2409116.
Atzeni, F, Talotta, R, Salaffi, F, Cassinotti, A, Varisco, V, Battellino, M, et al. Immunogenicity and autoimmunity during anti-TNF therapy. Autoimmun Rev. (2013) 12:703-8. doi: 10.1016/j.autrev.2012.10.021.
De Groot, AS, Scott, DW. Immunogenicity of protein therapeutics. Trends Immunol. (2007) 28:482-90. doi: 10.1016/j.it.2007.07.011.
de Vries, MK, Wolbink, GJ, Stapel, SO, de Groot, ER, Dijkmans, BA, Aarden, LA, et al. Inefficacy of infliximab in ankylosing spondylitis is correlated with antibody formation. Ann Rheum Dis. (2007) 66:133-4. doi: 10.1136/ard.2006.057745.
Hansel, TT, Kropshofer, H, Singer, T, Mitchell, JA, George, AJ. The safety and side effects of monoclonal antibodies. Nat Rev Drug Discov. (2010) 9:325-38. doi: 10.1038/nrd3003.
Hwang, WY, Foote, J. Immunogenicity of engineered antibodies. Methods. (2005) 36:3-10. doi: 10.1016/j.ymeth.2005.01.001.
Kabat EA, Te, Wu T, Bilofsky H. (U.S.) NI of H. Sequences of Immunoglobulin Chains: Tabulation Analysis of Amino Acid Sequences of Precursors, V-regions, C-regions, J-Chain BP-Microglobulins, 1979. Department of Health, Education, Welfare, Public Health Service, National Institutes of Health (1979).
Krieckaert CLM, Bartelds GM, Lems WF, Wolbink GJ. The effect of immunomodulators on the immunogenicity of TNF-blocking therapeutic monoclonal antibodies: a review. Arthritis Res Ther. 2010;12(5):217. doi: 10.1186/ar3147.
Liu L. Pharmacokinetics of monoclonal antibodies and Fc-fusion proteins. Protein Cell. 2018;9(1):15-32. doi: 10.1007/s13238-017-0408-4.
Ma J, Mo Y, Tang M, et al. Bispecific antibodies: from research to clinical application. Front Immunol 2021;12:626616. 10.3389/fimmu.2021.626616
Shankar G, Arkin S, Cocea L, Devanarayan V, Kirshner S, Kromminga A, Quarmby V, et al. Assessment and reporting of the clinical immunogenicity of therapeutic proteins and peptides-harmonized terminology and tactical recommendations. AAPS J. 2014;16(4):658-673. doi: 10.1208/s12248-014-9599-2.
Shields RL, Lai J, Keck R, O'Connell LY, Hong K, Meng YG, Weikert SH, Presta LG. Lack of fucose on human IgG1 N-linked oligosaccharide improves binding to human Fcgamma Rill and antibody-dependent cellular toxicity. J Biol Chem. 2002 Jul 26;277(30):26733-40.
Raju TS. Terminal sugars of Fc glycans influence antibody effector functions of IgGs. Curr Opin Immunol. 2008 Aug;20(4):471-8. doi: 10.1016/j.coi.2008.06.007. Epub 2008 Jul 17. PMID: 18606225.
Torre, B.G., Albericio, F. The pharmaceutical industry in 2023: an analysis of FDA drug approvals from the perspective of molecules. Molecules. 2024;29(3):585. doi: 10.3390/molecules29030585.
Vaughan, TJ, Williams, AJ, Pritchard, K, Osbourn, JK, Pope, AR, Earnshaw, JC, et al. Human antibodies with sub-nanomolar affinities isolated from a large non-immunized phage display library. Nat Biotechnol.(1996) 14:309-14. doi: 10.1038/nbt0396-309.
Walsh G, Walsh E. Biopharmaceutical benchmarks 2022. Nat Biotechnol. 2022;40(12):1722-1760. doi: 10.1038/s41587-022-01582-x
Wang Y-MC, Wang J, Hon YY, Zhou L, Fang L, Ahn HY. Evaluating and reporting the immunogenicity impacts for biological products-a clinical pharmacology perspective. APPS J. 2016;18(2):395-403. doi: 10.1208/s12248-015-9857-y.
Wang TT. (2019) Fc Mediated Activity of Antibodies. Edited by Ravetch JV and Nimmerjahn F. Switzerland: Springer Nature. Wei, J., Yang, Y, Wang, G., Liu, M. Current landscape and future directions of bispecific antibodies in cancer immunotherapy. Front Immunol 2022(13): 1035276. doi: 10.3389/fimmu.2022.1035276
Winter, G, Griffiths, AD, Hawkins, RE, Hoogenboom, HR. Making antibodies by phage display technology. Annu Rev Immunol. (1994) 12:433-55. doi: 10.1146/annurev.iy.12.040194.002245.
Yanai, H, Hanauer, SB. Assessing response and loss of response to biological therapies in IBD. Am J Gastroenterol. (2011) 106:685-98. doi: 10.1038/ajg.2011.103.
Zhou, Y, Penny, HL., Kroenke, MA., Bautista, B., Hainline, K., Chea LS., Parnes J., Mytych DT. Immunogenicity assessment of bispecific antibody-based immunotherapy in oncology. J Immunother Cancer 2022(10): e004225. doi: 10.1136/jitc-2021-004225.

## Claims

1. An antibody or antigen binding fragment thereof with reduced ADA binding comprising a modified amino acid sequence in the variable region wherein said amino acid sequence is modified to comprise one or more N-X-S and/or N-X-T sequence motifs wherein X is any amino acid other than proline.

2. The antibody or antigen binding fragment thereof according to claim 1 wherein a CDR1, 2 or 3 region or a region within 20, e.g. 10 amino acid residues of a framework region adjacent to a CDR1, 2 or 3 region is modified.

3. The antibody or antigen binding fragment thereof according to claim 1 or 2 wherein one or more amino acid residue located in a variable region is substituted with N, T or S.

4. The antibody or antigen binding fragment thereof according to claim 1 or 2 wherein a N-X-S/T sequence motif is inserted into an amino acid residue sequence.

5. The antibody or antigen binding fragment thereof according to claim 4 wherein a CDR1, 2 or 3 region or a region or within 1, 2, 3, 4 or 5 amino acid residues of a framework region adjacent to a CDR1, 2 or 3 region is modified.

6. The antibody or antigen binding fragment thereof according to a preceding claim wherein a CDR1, 2 or 3 region is modified.

7. The antibody or antigen binding fragment thereof according to a preceding claim wherein said one or more motif is located in an epitope for binding ADAs or in proximity thereto.

8. The antibody or antigen binding fragment thereof according to claim 3 wherein the one or more amino acid residue is substituted with N.

9. The antibody or antigen binding fragment thereof according to claim 3 wherein the one or more amino acid residue is substituted with T.

10. The antibody or antigen binding fragment thereof according to claim 3 wherein the one or more amino acid residue is replaced with S.

11. The antibody or antigen binding fragment thereof according to a preceding claim wherein said variable region is a heavy chain variable region or light chain variable region.

12. The antibody or antigen binding fragment thereof according to a preceding claim wherein said fragment is selected from a F(ab')2, Fab, Fv, scFv, heavy chain, light chain, variable heavy (VH), variable light (VL) chain, CDR region, single VH, VHH or VL domain, maxibodies, minibodies, intrabodies, diabodies, triabodies, tetrabodies, and bis-scFv, and polypeptides that contain at least a portion of an immunoglobulin that is sufficient to confer specific antigen binding to the polypeptide.

13. A pharmaceutical composition comprising an antibody or antigen binding fragment thereof according to any of claims 1 to 12 and a pharmaceutically acceptable excipient.

14. An antibody or antigen binding fragment thereof according to any of claims 1 to 12 or a pharmaceutical composition according to claim 13 for use in the treatment of cancer, inflammatory disease, autoimmune disease, hematology conditions, endocrine disorders, infectious diseases, pulmonary diseases, cardiovascular disease, neurological disorders, allergic diseases, metabolic disorders, ophthalmic diseases, bone and joint diseases, dermatological diseases or transplantation associated diseases.

15. A method for reducing the ADA response of an antibody or antigen binding fragment thereof with reduced immunogenicity comprising modifying the amino acid sequence of the variable region to comprise one or more N-X-S and/or N-X-T sequence motifs wherein X is any amino acid other than proline.
